# EUROPEAN PATENT APPLICATION

(11) **EP 4 194 560 A1**
(43) Date of publication of application: **14.06.2023**
(21) Application number: 21213084.3
(22) Date of filing: 08.12.2021
(51) Int. Cl.: C12P 21/00, C07K 14/37, C07K 14/39, C12N 9/90, C12N 15/80

(54) **IMPROVED PRODUCTION OF SECRETED PROTEINS IN FUNGAL CELLS**

(71) Applicant: European Molecular Biology Laboratory, 69117 Heidelberg (DE); Lesaffre et Compagnie, 75001 Paris (FR)
(72) Inventor: Steinmetz, Lars, 69115 Heidelberg (DE); Johansson, S. Andreas, 69120 Heidelberg (DE); Desfougeres, Thomas, 86130 Dissay (FR); Dulermo, Thierry, 59170 Croix (FR); Pignede, Georges, 59700 Marcq en Baroeul (FR)
(74) Representative: Krauss, Jan

(57) **Abstract**

The present invention relates to a yeast or filamentous fungal cell producing at least one secreted protein of interest, wherein said cell comprises at least one additional fungal gene showing increased expression and/or overexpression, showing reduced expression and/or inactivation, wherein said gene improves the production of the at least one secreted protein of interest. The present invention further relates to respective methods for production and uses of the yeast or filamentous fungal cells.

## Description

The present invention relates to a yeast or filamentous fungal cell producing at least one secreted protein of interest, wherein said cell comprises at least one additional fungal gene showing increased expression and/or overexpression, showing reduced expression and/or inactivation, wherein said gene improves the production of the at least one secreted protein of interest. The present invention further relates to respective methods for production and uses of the yeast or filamentous fungal cells.

### Background of the invention

The production of recombinant enzymes is growing rapidly and is estimated to generate several tens of billions of dollars (Martinez et al., 2012). Almost 60% of the enzymes used in detergents, the food industry and biofuel alcohol are recombinant enzymes, i.e. produced by an organism other than that of origin of the protein (COWAN, 1996). The expression of enzymes in a heterologous host allows (i) the production of enzymes of interest from slow growing or even non-cultivable organisms, (ii) the much higher production of the enzyme of interest, (iii ) the production of proteins from pathogenic or toxin-producing organisms, and (iv) the increase of the stability or activity of an enzyme by protein engineering (Falch, 1991; Demain and Vaishnav, 2009).

Many microorganisms, including filamentous fungi *(Aspergillus sp., Trichoderma sp.),* yeasts (for example *Pichia pastoris, Saccharomyces cerevisiae, Yarrowia lipolytica)* or bacteria (for example *Escherichia coli, Bacillus sp.),* are used to produce recombinant proteins (Demain and Vaishnav, 2009).

The production of recombinant proteins is dependent on the expression cassette (promoters and terminators used, signal sequence, codon bias), on the cellular machinery involved in the synthesis and degradation of proteins, intracellular trafficking and/or secretion, but also the energy level and/or redox of the cell as well as the culture conditions and the availability of nutrients (Zahrl et al., 2019).

Compared to other organisms conventionally used to produce recombinant proteins, *S*. *cerevisiae* has the advantage of rapid growth, easy manipulation both at the genetic level and at the level of production in bioreactors, and having Generally Recognized As Safe (GRAS) status. The production of a heterologous target protein in yeast host cells is further advantageous in that it allows the target proteins to be folded and secreted through the cellular secretory machinery.

Yeast is already widely used for many industrial applications (breadmaking, production of drinking alcohol and biofuels, etc. Parapouli *et al.,* 2020) where it may be advantageous to have it produce heterologous enzymes. For example, in the field of biofuel alcohol, the commercialized yeast strains of *S. cerevisiae* secrete enzymatic activities allowing the degradation of industrial mashes containing starch derivatives. This allows bioethanol manufacturers to limit their intake of exogenous enzymes and reduce their production costs.

US 2011-0129872A1 relates to a method for producing a recombinant protein, comprising culturing a yeast transformed with a recombinant gene construct comprising a yeast promoter, a gene coding a signal sequence and a gene coding a target protein; and also with one or more genes coding folding accessory protein selected from the group consisting of *PDI1* (protein disulfide isomerase 1), *SEC23* (secretory 23), *TRX2* (thioredoxin 2) *AHA1* (activator of heat shock protein 90 ATPase), and *SCJ1 (S. cerevisiae* DnaJ), followed by culturing the transformed yeast.

US 2013-0011875 relates to a method and the production of higher titers of recombinant protein in a modified yeast host cell, for example *Pichia pastoris,* wherein the modified yeast cell lacks vacuolar sorting activity or has decreased vacuolar sorting activity relative to an unmodified yeast host cell of the same species.

US 2014-0335622 discloses an expression vector for secreting a protein (Z) to be recovered or a fusion protein having the protein (Z) moiety therein; a method for producing a transformant using the expression vector; the transformant; and a method for producing a protein using the transformant. It is disclosed that co-expression of a foreign secretory protein with *PDI1* increases the secretory production amount.

US 2016-0186192 describes a method for producing a desired protein comprising: (a) providing a host cell comprising a first recombinant gene encoding a protein comprising the sequence of a first chaperone protein, a second recombinant gene encoding a protein comprising the sequence of a second chaperone protein and a third gene, such as a third recombinant gene, encoding a desired protein (such as a desired heterologous protein), wherein the first and second chaperones are different; and (b) culturing the host cell in a culture medium to obtain expression of the first, second and third genes.

US 2018-0022785 claims a method for producing a heterologous protein, said method comprising: culturing a *Saccharomyces cerevisiae* yeast host cell or a culture thereof to produce the heterologous protein, wherein said *Saccharomyces cerevisiae* yeast host cell comprises a modified Not4 protein, and wherein said heterologous protein is an albumin, or a variant, fragment and/or fusion thereof.

Eun Jung Thak et al. (in: Yeast synthetic biology for designed cell factories producing secretory recombinant proteins, FEMS Yeast Research, Volume 20, Issue 2, March 2020, foaa009, https://doi.org/10.1093/femsyr/foaa009) disclose that yeasts are prominent hosts for the production of recombinant proteins from industrial enzymes to therapeutic proteins. Particularly, the similarity of protein secretion pathways between these unicellular eukaryotic microorganisms and higher eukaryotic organisms has made them a preferential host to produce secretory recombinant proteins. However, there are several bottlenecks, in terms of quality and quantity, restricting their use as secretory recombinant protein production hosts. They discuss recent developments in synthetic biology approaches to constructing yeast cell factories endowed with enhanced capacities of protein folding and secretion as well as designed targeted post-translational modification process functions, and focus on the new genetic tools for optimizing secretory protein expression, such as codon-optimized synthetic genes, combinatory synthetic signal peptides and copy number-controllable integration systems, and the advanced cellular engineering strategies, including endoplasmic reticulum and protein trafficking pathway engineering, synthetic glycosylation, and cell wall engineering, for improving the quality and yield of secretory recombinant proteins.

Zihe Liu, etal. (in: Improved Production of a Heterologous Amylase in Saccharomyces cerevisiae by Inverse Metabolic Engineering, Applied and Environmental Microbiology Aug 2014, 80 (17) 5542-5550; DOI: 10.1128/AEM.00712-14) disclose that the increasing demand for industrial enzymes and biopharmaceutical proteins relies on robust production hosts with high protein yield and productivity. Being one of the best-studied model organisms and capable of performing posttranslational modifications, the yeast Saccharomyces cerevisiae is widely used as a cell factory for recombinant protein production. However, many recombinant proteins are produced at only 1% (or less) of the theoretical capacity due to the complexity of the secretory pathway, which has not been fully exploited. They applied the concept of inverse metabolic engineering to identify novel targets for improving protein secretion. Screening that combined UV-random mutagenesis and selection for growth on starch was performed to find mutant strains producing heterologous amylase 5-fold above the level produced by the reference strain. Genomic mutations that could be associated with higher amylase secretion were identified through whole-genome sequencing. Several single-point mutations, including an S196I point mutation in the VTA1 gene coding for a protein involved in vacuolar sorting, were evaluated by introducing these to the starting strain. By applying this modification alone, the amylase secretion could be improved by 35%. As a complement to the identification of genomic variants, transcriptome analysis was also performed in order to understand on a global level the transcriptional changes associated with the improved amylase production caused by UV mutagenesis.

Huang, M., et al. (in: Efficient protein production by yeast requires global tuning of metabolism. Nat Commun 8, 1131 (2017). https://doi.org/10.1038/s41467-017-00999-2) describe that the biotech industry relies on cell factories for production of pharmaceutical proteins, of which several are among the top-selling medicines. There is, therefore, considerable interest in improving the efficiency of protein production by cell factories. Protein secretion involves numerous intracellular processes with many underlying mechanisms still remaining unclear. They used RNA-seq to study the genome-wide transcriptional response to protein secretion in mutant yeast strains, and find that many cellular processes have to be attuned to support efficient protein secretion. In particular, altered energy metabolism resulting in reduced respiration and increased fermentation, as well as balancing of amino-acid biosynthesis and reduced thiamine biosynthesis seem to be particularly important. They confirmed their findings by inverse engineering and physiological characterization and show that by tuning metabolism cells are able to efficiently secrete recombinant proteins.

Huang M, et al. (In: Microfluidic screening and whole-genome sequencing identifies mutations associated with improved protein secretion by yeast. Proc Natl Acad Sci U S A. 2015 Aug 25;112(34):E4689-96. doi: 10.1073/pnas.1506460112. Epub 2015 Aug 10. PMID: 26261321; PMCID: PMC4553813) disclose that there is an increasing demand for biotech-based production of recombinant proteins for use as pharmaceuticals in the food and feed industry and in industrial applications, that the yeast *Saccharomyces cerevisiae* is among preferred cell factories for recombinant protein production, and there is increasing interest in improving its protein secretion capacity. Due to the complexity of the secretory machinery in eukaryotic cells, it is said to be difficult to apply rational engineering for construction of improved strains. They used high-throughput microfluidics for the screening of yeast libraries, generated by UV mutagenesis. Several screening and sorting rounds resulted in the selection of eight yeast clones with significantly improved secretion of recombinant α-amylase. Efficient secretion was genetically stable in the selected clones. They performed whole-genome sequencing of the eight clones and identified 330 mutations in total. Gene ontology analysis of mutated genes revealed many biological processes, including some that had not been identified before in the context of protein secretion. Mutated genes identified are disclosed to be potentially used for reverse metabolic engineering, with the objective to construct efficient cell factories for protein secretion. The combined use of microfluidics screening and whole-genome sequencing to map the mutations associated with the improved phenotype can easily be adapted for other products and cell types to identify novel engineering targets, and this approach could broadly facilitate design of novel cell factories.

Finally, Huang M, et al. (in: Engineering the protein secretory pathway of Saccharomyces cerevisiae enables improved protein production. Proc Natl Acad Sci U S A. 2018 Nov 20;115(47):E11025-E11032. doi: 10.1073/pnas.1809921115. Epub 2018 Nov 5. PMID: 30397111; PMCID: PMC6255153) describe that baker's yeast Saccharomyces cerevisiae is one of the most important and widely used cell factories for recombinant protein production. Many strategies have been applied to engineer this yeast for improving its protein production capacity, but productivity is still relatively low, and with increasing market demand, it is important to identify new gene targets, especially targets that have synergistic effects with previously identified targets. Despite improved protein production, previous studies rarely focused on processes associated with intracellular protein retention. They identified genetic modifications involved in the secretory and trafficking pathways, the histone deacetylase complex, and carbohydrate metabolic processes as targets for improving protein secretion in yeast. Especially modifications of endosome-to-Golgi trafficking was found to effectively reduce protein retention besides increasing protein secretion. Through combinatorial genetic manipulations of several of the newly identified gene targets, they enhanced the protein production capacity of yeast by more than fivefold, and the best engineered strains could produce 2.5 g/L of a fungal α-amylase with less than 10% of the recombinant protein retained within the cells, using fed-batch cultivation.

Cryptic unstable transcripts (CUTs) are a subset of non-coding RNAs (ncRNAs) that are produced from intergenic and intragenic regions. Additionally, stable uncharacterized transcripts, or SUTs, have also been detected in cells and bear many similarities to CUTs but are not degraded through the same pathways.

Genetic engineering strategies to overcome bottlenecks in the yeast protein secretion pathway have to consider that protein secretion in yeast involves multiple complex steps, such as protein translocation, folding, post-translational modification and vesicle trafficking between several membrane organelles and plasma membranes. The secretion of proteins synthesized inside cells can be hampered by low secretion efficiency, abnormal post-translational modifications, retention within the secretion pathway or the cell wall space as a cell-associated form. The development of engineering strategies targeted to each step of the secretion pathway in a modular fashion is required in order to design cell factories producing secretory recombinant proteins. Today, despite its obvious qualities, *S. cerevisiae* remains relatively limited in its ability to secrete proteins compared to organisms such as filamentous fungi or *P. pastoris* (Demain and Vaishnav, 2009). It is therefore an object of the present invention to provide new factors to improve recombinant protein production and secretion in yeast. Other objects and advantages will become apparent to the person of skill when studying the present description of the present invention.

In a first aspect of the present invention, the above object is solved by providing a yeast or filamentous fungal cell producing at least one secreted protein of interest, wherein said cell comprises at least one fungal gene selected from the group consisting of MIC19, TOM22, NKP1, DML1, CUT859, GAL80, APM3, COQ10, BLM10, MDH1, VHS2, ASA1, TRP4, YPS7, CUT824, YOR318C, PRM7, ERV46, FIT2, GPM3, CUT892, SRN2, SUT643, CUT461, THR4, GMH1, SOL1, NAB6, YPR148C, ALP1, CUT097, ATG33, YOR316C-A, SOG2, MCM6, SUT230, SUT419, TIF11, TAF5, PHO91, AIM32, ENO2, UBA2, PUS5, ERG1, SUT311, KSS1, MRP10, CUT598, CUT188, YOR238W, EMW1, BNA7, SNR63, CCT3, PRY2, MAL11, KRS1 RAI1, SUT784, YPR148C, YEL1, CUT832, NMA2, VPS27, SUT428, PEX29, YLR446W, WBP1, AVT2, CUT854, TRM10, SLX9, YPL077C, PET122, TFG2, PUN1, CUT152, AIR2, CUT571, RPS26B, RRT6, RPC19, URA3, YGR045C, SMC3, PNG1, THI6, MEU1, CUT239, NSE4, SUT074, AAH1, RMD5, CUT607, ACS1, MNN1, ARH1, YHR140W, CET1, RRB1, YLR342W-A, RPS22B, CHS5, YIL165C, SUT093, LPX1, NCA3, EFG1, NBP35, CUT765, MSL1, SCD6, ATG42, CHS6, COQ2, RPO31, MKK1, HED1, PBP2, BET5, CUT678, YGR021W, SUT474, YGL159W, IRC21, VHR1, SPP1, PRP43, ZRT1, YLR041W, SUT711, COX18, CBP6, SUT575, CLG1, CUT213, QCR10, SNR3, MSS2, CUT505, YOS1, SUT073, UTP21, ACA1, CUT632, RIP1, HUL5, CUT727, RPL35B, CUT184, CUT420, YFL041W-A, SUT460, ATG10, MFA1, UGX2, TRK2, CUT704, SUT083, TRE1, RVS161, LEA1, EBP2, THI80, CTI6, CUT322, XPT1, MRPL35, YPL025C, SUT737, PGA2, ULP2, MRX16, EST1, NUP100, IES3, ATG39, YMR084W, SUT428, YPL119C-A, MIN8, CUT490, SUT287, KEL3, SUT678, SEC3, SOL4, SIS2, CUT915, RRP3, ESA1, PCL8, TRX3, YKL115C, EMP65, ZDS1, CUT167, SOD1, UBR2, LSP1, SNR81, RGD3, YTP1, SMY2, CUT449, FIN1, YKL106C-A, YAR019W-A, CCH1, AYR1, SUT573, VNX1, FOL3, SUT511, GIS4, CUT743, RPL24A, HMT1, SUT333, SPP2, SUT128, SMC6, PHR1, RPS15, CUT642, GYP7, tK(CUU)K, CUT896, SLM5, CUT586, CUT158, and RRP12, wherein said at least one fungal gene shows increased expression and/or overexpression, and/or wherein said cell comprises at least one fungal gene selected from the group consisting of TLG2, CUT901, ATG33, THR4, YDR262W, CMC1, MRP17, YPT52, CUT312, MRPS5, RDR1, DAL7, RPL20A, YBR137W, RPL36B, YEL008C-A, RAX1, CUT729, INP51, UBP8, CUT258, YLR342W-A, SUT568, PEX7, MSD1, CUT136, TIM10, CUT361, snR51, TAL1, RIP1, MRP10, SUT078, MRP51, GLO3, EHD3, HER1, NMA111, PBP4, MFB1, IKI3, NDL1, SUT433, YOR238W, SUT750, QDR2, RDI1, SUT014, CUT437, MSC6, SUT497, YCR051W, MRPL33, RPL14A, TRM7, RNH202, RTC5, SUT027, CDC5, SUT729, YOR131C, CUT665, GLG2, SUT268, SUT705, MED4, RCR2, EFB1, RXT2, KGD1, TUP1, RNH203, YDR338C, SED1, CUT522, HIS2, SUT145, MET17, APC4, NKP2, MKK2, NDC1, PET100, NIP7, VHT1, SUT685, BNI5, SNA3, EGH1, MRP4, POB3, PIB2, SUT317, and NTO1, wherein said at least one fungal gene shows reduced expression and/or inactivation.

Preferably, said cell comprises at least one fungal gene selected from the group consisting of MIC19, TOM22, NKP1, DML1, CUT859, GAL80, APM3, COQ10, BLM10, MDH1, EMW1, BNA7, SNR63, CCT3, PRY2, MAL11, KRS1, RAI1, SUT784, YPR148C, YEL1, CUT832, NMA2, VPS27, SUT428, PEX29, YLR446W, and WBP1, wherein said at least one fungal gene shows increased expression and/or overexpression, and/or wherein said cell comprises at least one fungal gene selected from the group consisting of TLG2, CUT901, ATG33, THR4, NDC1, PET100, NIP7, VHT1, and SUT685, wherein said at least one fungal gene shows reduced expression and/or inactivation..

More preferred is the yeast or filamentous fungal cell according to the present invention, wherein said genes or SUTs or CUTs are furthermore selected from the group of genes or SUTs or CUTs having a value of logFC/FDR of more than 200, more preferred of more than 300, and most preferred of more than 500, based on the values as determined herein.

More preferred is the yeast or filamentous fungal cell according to the present invention, further comprising a fungal gene selected from the group consisting of THR4, MRP10, RIP1, YLR342W-A, ATG33, and YOR238W, either showing an increased expression and/or overexpression or reduced expression and/or inactivation, depending on the experimental conditions, such as, without wanting to be bound by theory, for example, the impact of CRISPRa and CRISPRi on gene expression due to the position of the gRNA in the promoting region.

Even more preferred is the yeast or filamentous fungal cell according to the present invention, further comprising the fungal gene *PDI1,* showing an increased expression and/or overexpression.

Advantageously, the yeast or filamentous fungal cell according to the present invention produces the at least one secreted protein to about 30% or more, or about 40% or more, preferably about 50% or more, more preferably to about 75% or more, when compared to a control yeast or filamentous fungal cell.

In a second aspect of the present invention, the above object is solved by a method for producing a secreted protein in a yeast or filamentous fungal cell, comprising the steps of i) providing a yeast or filamentous fungal cell producing at least one secreted protein of interest according to the present invention, ii) culturing said yeast or filamentous fungal cell in suitable culture medium, and iii) isolating said secreted protein from aid culture medium. Preferred is the method according to the present invention, further comprising suitably inducing the increased expression and/or overexpression or reduced expression and/or inactivation of the at least one fungal gene.

Further preferred is the method according to the present invention, wherein about 30% or more, or about 40% or more, preferably about 50% or more, more preferably to about 75% or more of said at least one secreted protein is produced, when compared to the production of a control yeast or filamentous fungal cell.

In a third aspect of the present invention, the above object is solved by a method for producing a yeast or filamentous fungal cell producing at least one secreted protein of interest according to the present invention, comprising introducing into said cell producing at least one secreted protein of interest at least one fungal gene selected from the group consisting of MIC19, TOM22, NKP1, DML1, CUT859, GAL80, APM3, COQ10, BLM10, MDH1, VHS2, ASA1, TRP4, YPS7, CUT824, YOR318C, PRM7, ERV46, FIT2, GPM3, CUT892, SRN2, SUT643, CUT461, THR4, GMH1, SOL1, NAB6, YPR148C, ALP1, CUT097, ATG33, YOR316C-A, SOG2, MCM6, SUT230, SUT419, TIF11, TAF5, PHO91, AIM32, ENO2, UBA2, PUS5, ERG1, SUT311, KSS1, MRP10, CUT598, CUT188, YOR238W, EMW1, BNA7, SNR63, CCT3, PRY2, MAL11, KRS1 RAI1, SUT784, YPR148C, YEL1, CUT832, NMA2, VPS27, SUT428, PEX29, YLR446W, WBP1, AVT2, CUT854, TRM10, SLX9, YPL077C, PET122, TFG2, PUN1, CUT152, AIR2, CUT571, RPS26B, RRT6, RPC19, URA3, YGR045C, SMC3, PNG1, THI6, MEU1, CUT239, NSE4, SUT074, AAH1, RMD5, CUT607, ACS1, MNN1, ARH1, YHR140W, CET1, RRB1, YLR342W-A, RPS22B, CHS5, YIL165C, SUT093, LPX1, NCA3, EFG1, NBP35, CUT765, MSL1, SCD6, ATG42, CHS6, COQ2, RPO31, MKK1, HED1, PBP2, BET5, CUT678, YGR021W, SUT474, YGL159W, IRC21, VHR1, SPP1, PRP43, ZRT1, YLR041W, SUT711, COX18, CBP6, SUT575, CLG1, CUT213, QCR10, SNR3, MSS2, CUT505, YOS1, SUT073, UTP21, ACA1, CUT632, RIP1, HUL5, CUT727, RPL35B, CUT184, CUT420, YFL041W-A, SUT460, ATG10, MFA1, UGX2, TRK2, CUT704, SUT083, TRE1, RVS161, LEA1, EBP2, THI80, CTI6, CUT322, XPT1, MRPL35, YPL025C, SUT737, PGA2, ULP2, MRX16, EST1, NUP100, IES3, ATG39, YMR084W, SUT428, YPL119C-A, MIN8, CUT490, SUT287, KEL3, SUT678, SEC3, SOL4, SIS2, CUT915, RRP3, ESA1, PCL8, TRX3, YKL115C, EMP65, ZDS1, CUT167, SOD1, UBR2, LSP1, SNR81, RGD3, YTP1, SMY2, CUT449, FIN1, YKL106C-A, YAR019W-A, CCH1, AYR1, SUT573, VNX1, FOL3, SUT511, GIS4, CUT743, RPL24A, HMT1, SUT333, SPP2, SUT128, SMC6, PHR1, RPS15, CUT642, GYP7, tK(CUU)K, CUT896, SLM5, CUT586, CUT158, and RRP12, wherein said at least one fungal gene shows increased expression and/or overexpression, and/or wherein said cell comprises at least one fungal gene selected from the group consisting of TLG2, CUT901, ATG33, THR4, YDR262W, CMC1, MRP17, YPT52, CUT312, MRPS5, RDR1, DAL7, RPL20A, YBR137W, RPL36B, YEL008C-A, RAX1, INP51, CUT729, UBP8, CUT258, YLR342W-A, SUT568, PEX7, MSD1, CUT136, TIM10, CUT361, snR51, TAL1, RIP1, MRP10, SUT078, MRP51, GLO3, EHD3, HER1, NMA111, PBP4, MFB1, IKI3, NDL1, SUT433, YOR238W, SUT750, QDR2, RDI1, SUT014, CUT437, MSC6, SUT497, YCR051W, MRPL33, RPL14A, TRM7, RNH202, RTC5, SUT027, CDC5, SUT729, YOR131C, CUT665, GLG2, SUT268, SUT705, MED4, RCR2, EFB1, RXT2, KGD1, TUP1, RNH203, YDR338C, SED1, CUT522, HIS2, SUT145, MET17, APC4, NKP2, MKK2, NDC1, PET100, NIP7, VHT1, SUT685, BNI5, SNA3, EGH1, MRP4, POB3, PIB2, SUT317, and NTO1, wherein said at least one fungal gene shows reduced expression and/or inactivation..

Preferred is a method of the present invention for producing a yeast or filamentous fungal cell producing at least one secreted protein of interest according to the present invention, comprising introducing into said cell producing at least one secreted protein of interest at least one fungal gene selected from the group consisting of MIC19, TOM22, NKP1, DML1, CUT859, GAL80, APM3, COQ10, BLM10, MDH1, EMW1, BNA7, SNR63, CCT3, PRY2, MAL11, KRS1, RAI1, SUT784, YPR148C, YEL1, CUT832, NMA2, VPS27, SUT428, PEX29, YLR446W, and WBP1, wherein said at least one fungal gene shows increased expression and/or overexpression, and/or wherein said cell comprises at least one fungal gene selected from the group consisting of TLG2, CUT901, ATG33, THR4, NDC1, PET100, NIP7, VHT1, and SUT685, wherein said at least one fungal gene shows reduced expression and/or inactivation.

Furthermore, the method according to the invention may include further introducing into said cell a fungal gene selected from the group consisting of THR4, MRP10, RIP1, YLR342W-A, ATG33, and YOR238W, either showing an increased expression and/or overexpression or reduced expression and/or inactivation, depending on the experimental conditions. Furthermore, the method may include further introducing into said cell the fungal gene *PDI1,* showing an increased expression and/or overexpression.

In a fourth aspect of the present invention, the above object is solved by the use of a yeast or filamentous fungal cell according to the present invention for producing at least one secreted protein of interest.

As mentioned above, the analysis of UV *S. cerevisiae* mutants expressing an α-amylase has revealed improved strains for secretion (Huang et al., 2015; Huang et al., 2018). Coupling microfluidics with a phenotypic screening using a starch complexed with BODIPY (which becomes fluorescent when it is released), the authors had selected the mutants secreting the most enzyme into the extracellular medium. The sequencing of eight hypersecretory clones (x1.5 to x6) revealed 330 mutations potentially involved in improving α-amylase production and secretion (Huang et al., 2015). A more in-depth analysis led to the identification of - amongst others as disclosed herein - a role of the known *PDI1* gene in the production and secretion of α-amylase in *S. cerevisiae.*

The purpose of the present invention was to discover new factors and genes involved in protein secretion in order to improve protein production and secretion, as exemplified in the industrial Ethanol Red ^{®} strain of *S. cerevisiae.*

As mentioned above, in the first aspect of the present invention, a yeast or filamentous fungal cell is provided that produces at least one secreted protein of interest. In addition, the cell comprises at least one fungal gene selected from the group consisting of MIC19, TOM22, NKP1, DML1, CUT859, GAL80, APM3, COQ10, BLM10, MDH1, VHS2, ASA1, TRP4, YPS7, CUT824, YOR318C, PRM7, ERV46, FIT2, GPM3, CUT892, SRN2, SUT643, CUT461, THR4, GMH1, SOL1, NAB6, YPR148C, ALP1, CUT097, ATG33, YOR316C-A, SOG2, MCM6, SUT230, SUT419, TIF11, TAF5, PHO91, AIM32, ENO2, UBA2, PUS5, ERG1, SUT311, KSS1, MRP10, CUT598, CUT188, YOR238W, EMW1, BNA7, SNR63, CCT3, PRY2, MAL11, KRS1 RAI1, SUT784, YPR148C, YEL1, CUT832, NMA2, VPS27, SUT428, PEX29, YLR446W, WBP1, AVT2, CUT854, TRM10, SLX9, YPL077C, PET122, TFG2, PUN1, CUT152, AIR2, CUT571, RPS26B, RRT6, RPC19, URA3, YGR045C, SMC3, PNG1, THI6, MEU1, CUT239, NSE4, SUT074, AAH1, RMD5, CUT607, ACS1, MNN1, ARH1, YHR140W, CET1, RRB1, YLR342W-A, RPS22B, CHS5, YIL165C, SUT093, LPX1, NCA3, EFG1, NBP35, CUT765, MSL1, SCD6, ATG42, CHS6, COQ2, RPO31, MKK1, HED1, PBP2, BET5, CUT678, YGR021W, SUT474, YGL159W, IRC21, VHR1, SPP1, PRP43, ZRT1, YLR041W, SUT711, COX18, CBP6, SUT575, CLG1, CUT213, QCR10, SNR3, MSS2, CUT505, YOS1, SUT073, UTP21, ACA1, CUT632, RIP1, HUL5, CUT727, RPL35B, CUT184, CUT420, YFL041W-A, SUT460, ATG10, MFA1, UGX2, TRK2, CUT704, SUT083, TRE1, RVS161, LEA1, EBP2, THI80, CTI6, CUT322, XPT1, MRPL35, YPL025C, SUT737, PGA2, ULP2, MRX16, EST1, NUP100, IES3, ATG39, YMR084W, SUT428, YPL119C-A, MIN8, CUT490, SUT287, KEL3, SUT678, SEC3, SOL4, SIS2, CUT915, RRP3, ESA1, PCL8, TRX3, YKL115C, EMP65, ZDS1, CUT167, SOD1, UBR2, LSP1, SNR81, RGD3, YTP1, SMY2, CUT449, FIN1, YKL106C-A, YAR019W-A, CCH1, AYR1, SUT573, VNX1, FOL3, SUT511, GIS4, CUT743, RPL24A, HMT1, SUT333, SPP2, SUT128, SMC6, PHR1, RPS15, CUT642, GYP7, tK(CUU)K, CUT896, SLM5, CUT586, CUT158, and RRP12, wherein these at least one fungal gene shows increased expression and/or overexpression. In the context of the present invention, the terms "increased expression" or "overexpression" indicate that the amount of protein as produced by the cell is higher when compared to the expression in a control cell showing normal, unaltered or baseline expression. The change in expression can be achieved in any suitable way, and examples include mutated promotors, cloning of the gene under the control of a heterologous "strong" promotor, either inducible or constitutive, codon optimization, and mutations that stabilize the structure of the protein, and the like. Alternatively or in addition, the cell comprises at least one fungal gene selected from the group consisting of TLG2, CUT901, ATG33, THR4, YDR262W, CMC1, MRP17, YPT52, CUT312, MRPS5, RDR1, DAL7, RPL20A, YBR137W, RPL36B, YEL008C-A, RAX1, INP51, CUT729, UBP8, CUT258, YLR342W-A, SUT568, PEX7, MSD1, CUT136, TIM10, CUT361, snR51, TAL1, RIP1, MRP10, SUT078, MRP51, GLO3, EHD3, HER1, NMA111, PBP4, MFB1, IKI3, NDL1, SUT433, YOR238W, SUT750, QDR2, RDI1, SUT014, CUT437, MSC6, SUT497, YCR051W, MRPL33, RPL14A, TRM7, RNH202, RTC5, SUT027, CDC5, SUT729, YOR131C, CUT665, GLG2, SUT268, SUT705, MED4, RCR2, EFB1, RXT2, KGD1, TUP1, RNH203, YDR338C, SED1, CUT522, HIS2, SUT145, MET17, APC4, NKP2, MKK2, NDC1, PET100, NIP7, VHT1, SUT685, BNI5, SNA3, EGH1, MRP4, POB3, PIB2, SUT317, and NTO1, wherein said at least one fungal gene shows reduced expression and/or inactivation, wherein said at least one fungal gene shows reduced expression and/or inactivation. In the context of the present invention, the terms "reduced expression" or "inactivation" indicate that the amount of protein as produced by the cell is lower when compared to the expression in a control cell showing normal, unaltered or baseline expression. The change in expression can be achieved in any suitable way, and examples include mutated promotors, cloning of the gene under the control of a heterologous "weak" promotor, either inducible or constitutive, codon changes, and mutations that de-stabilize the structure of the protein, and the like.

Preferably, said yeast or filamentous fungal cell as provided comprises at least one fungal gene selected from the group consisting of MIC19, TOM22, NKP1, DML1, CUT859, GAL80, APM3, COQ10, BLM10, MDH1, EMW1, BNA7, SNR63, CCT3, PRY2, MAL11, KRS1, RAI1, SUT784, YPR148C, YEL1, CUT832, NMA2, VPS27, SUT428, PEX29, YLR446W, and WBP1, wherein said at least one fungal gene shows increased expression and/or overexpression, and/or wherein said cell comprises at least one fungal gene selected from the group consisting of TLG2, CUT901, ATG33, THR4, NDC1, PET100, NIP7, VHT1, and SUT685, wherein said at least one fungal gene shows reduced expression and/or inactivation.

More preferred is the yeast or filamentous fungal cell according to the present invention, wherein said genes or SUTs or CUTs are furthermore selected from the group of genes or SUTs or CUTs having a value of logFC/FDR of more than 200, more preferred of more than 300, and most preferred of more than 500, based on the values as determined herein.

More preferred is the yeast or filamentous fungal cell according to the present invention, further comprising a fungal gene selected from the group consisting of THR4, MRP10, RIP1, YLR342W-A, ATG33, and YOR238W, either showing an increased expression and/or overexpression or reduced expression and/or inactivation, depending on the experimental conditions.

Even more preferred is the yeast or filamentous fungal cell according to the present invention, further comprising the fungal gene *PDI1,* showing an increased expression and/or overexpression.

It is expected that a combination of genes as mentioned herein can lead to an even further increased production of the protein of interest, even having synergistic effects. Examples for these combinations are all of *TLG2, YDR262W,* and *TRM10*, optionally further comprising *PDI1.* Other examples are *ATG33* and *MRP10,* NDC1 and *TRM10*, or *PRY2,* and *TOM22,* again each pair optionally further comprising *PDI1.*

Most preferred are either *AVT2*, *PRY2, SUT074, BNA7, TOM22* or *TRM10.* The overexpression of *AVT2, TRM10, PRY2, SUT074, BNA7,* or *TOM22,* and the inactivation of *INP51* is further preferred. Further examples are TLG2, CUT901, ATG33, THR4, YDR262W, and CMC1, optionally further comprising *PDI1.*

The fungal gene(s) and/or SUTs or CUTs as used are preferably derived from *S. cerevisiae,* or a related yeast. The fungal gene(s) and/or SUTs or CUTs and their reference numbers are according to the Saccharomyces Genome Database (*SGD*) (https://www.yeastgenome.org/), as of November 15, 2021. Related genes that may be used as well encode for proteins sharing the same biological effect (increased secretion) in the yeast or filamentous fungal cell with the genes as above, and/or have an amino acid identity of about 80% or more, preferably about 90% or more, more preferably about 95% or more with the polypeptide sequence as encoded by a genes as above.

Advantageously, preferably the yeast or filamentous fungal cell according to the present invention produces the at least one secreted protein to about 30% or more or 40% or more, preferably about 50% or more, more preferably to about 75% or more, when compared to a control yeast or filamentous fungal cell, preferably one that does not contain a gene as mentioned above leading to increased secretion of the protein of interest.

As the protein of interest, any protein can be chosen that can be suitably produced by the yeast or filamentous fungal cell according to the present invention, e.g. expressed, folded, glycosylated and/or secreted. The gene of the protein of interest can be codon optimized, and preferably show an increased expression and/or overexpression, as explained above for the fungal gene according to the present invention. Examples of preferred proteins of interest are human serum albumin (HSA), amylase, human insulin, and components of hepatitis vaccines, human papillomavirus (HPV) vaccines, interferon(s), or epidermal growth factor (hEGF), and proteins used in food production, such as cellulase, glucoamylase, xylanase, and the like.

In order to identify new genes involved in the production and secretion of recombinant and heterologous proteins in yeast or filamentous fungal cells, such as *S. cerevisiae,* the inventors have developed CRISPRi and CRISPRa libraries allowing the overexpression or the repression of all genes as well as previously identified Stable Unannotated Transcripts (SUT's) and (Cryptic Unstable Transcripts CUT's) of this yeast (see Xu, Z. et al. Bidirectional promoters generate pervasive transcription in yeast. Nature 457, 1033-1037 (2009)). These libraries utilize an inactivated Cas9 (dCas9) able to bind DNA at the CRISPR site but unable to cleave the DNA molecule, fused to a transcriptional activation (CRISPRa) (e.g. the VP64-p65-Rta (VPR) tripartite activation domain described in Chavez, A. et al. Highly efficient Cas9-mediated transcriptional programming. Nat Methods 12, 326-328 (2015)) or repression domain (CRISPRi) (Dominguez *et al.,* 2015).

The industrial Ethanol Red^{®} (ER) yeast strain overexpressing an α-amylase (*Amy6* from *A. niger*) was used as a model for the present invention (Lesaffre, Marcq-en-Barœul, France). A 40,890 gRNA library targeting the promoters of 7,247 yeast genes, SUT's and CUT's at an average of 5.8 positions per gene, SUT or CUT was developed and cloned into replicative vectors allowing their expression as well as the expression of dCas9-VP64-p65-Rta (CRISPRa) or dCas9-Mxi1 (CRISPRi). The ER + α-amylase strain was then transformed using the CRISPRa and CRISPRi libraries, and the cell population as obtained was screened by microfluidics on the basis of its capacity to degrade a starch substrate labelled with BODIPY FL dye which fluoresces in green when the starch is degraded by α-amylase (e.g. EnzChek^{®} Ultra Amylase Assay Kit: https://www.thermofisher.com/order/catalog/product/E33651#/E33651).

Clones presenting high fluorescence were sorted, and gRNA regions from replicative vectors were analyzed by Illumina sequencing. Data analysis revealed that 320 activated or repressed genes favor α-amylase secretion. These genes were manually selected further, and the genes MIC19, TOM22, NKP1, DML1, CUT859, GAL80, APM3, COQ10, BLM10, MDH1, VHS2, ASA1, TRP4, YPS7, CUT824, YOR318C, PRM7, ERV46, FIT2, GPM3, CUT892, SRN2, SUT643, CUT461, THR4, GMH1, SOL1, NAB6, YPR148C, ALP1, CUT097, ATG33, YOR316C-A, SOG2, MCM6, SUT230, SUT419, TIF11, TAF5, PHO91, AIM32, ENO2, UBA2, PUS5, ERG1, SUT311, KSS1, MRP10, CUT598, CUT188, YOR238W, EMW1, BNA7, SNR63, CCT3, PRY2, MAL11, KRS1 RAI1, SUT784, YPR148C, YEL1, CUT832, NMA2, VPS27, SUT428, PEX29, YLR446W, WBP1, AVT2, CUT854, TRM10, SLX9, YPL077C, PET122, TFG2, PUN1, CUT152, AIR2, CUT571, RPS26B, RRT6, RPC19, URA3, YGR045C, SMC3, PNG1, THI6, MEU1, CUT239, NSE4, SUT074, AAH1, RMD5, CUT607, ACS1, MNN1, ARH1, YHR140W, CET1, RRB1, YLR342W-A, RPS22B, CHS5, YIL165C, SUT093, LPX1, NCA3, EFG1, NBP35, CUT765, MSL1, SCD6, ATG42, CHS6, COQ2, RPO31, MKK1, HED1, PBP2, BET5, CUT678, YGR021W, SUT474, YGL159W, IRC21, VHR1, SPP1, PRP43, ZRT1, YLR041W, SUT711, COX18, CBP6, SUT575, CLG1, CUT213, QCR10, SNR3, MSS2, CUT505, YOS1, SUT073, UTP21, ACA1, CUT632, RIP1, HUL5, CUT727, RPL35B, CUT184, CUT420, YFL041W-A, SUT460, ATG10, MFA1, UGX2, TRK2, CUT704, SUT083, TRE1, RVS161, LEA1, EBP2, THI80, CTI6, CUT322, XPT1, MRPL35, YPL025C, SUT737, PGA2, ULP2, MRX16, EST1, NUP100, IES3, ATG39, YMR084W, SUT428, YPL119C-A, MIN8, CUT490, SUT287, KEL3, SUT678, SEC3, SOL4, SIS2, CUT915, RRP3, ESA1, PCL8, TRX3, YKL115C, EMP65, ZDS1, CUT167, SOD1, UBR2, LSP1, SNR81, RGD3, YTP1, SMY2, CUT449, FIN1, YKL106C-A, YAR019W-A, CCH1, AYR1, SUT573, VNX1, FOL3, SUT511, GIS4, CUT743, RPL24A, HMT1, SUT333, SPP2, SUT128, SMC6, PHR1, RPS15, CUT642, GYP7, tK(CUU)K, CUT896, SLM5, CUT586, CUT158, and RRP12, were overexpressed using common techniques (integration of overexpression cassette into the genome and/or overexpression through a replicative plasmid), and genes TLG2, CUT901, ATG33, THR4, YDR262W, CMC1, MRP17, YPT52, CUT312, MRPS5, RDR1, DAL7, RPL20A, YBR137W, RPL36B, YEL008C-A, RAX1, INP51, CUT729, UBP8, CUT258, YLR342W-A, SUT568, PEX7, MSD1, CUT136, TIM10, CUT361, snR51, TAL1, RIP1, MRP10, SUT078, MRP51, GLO3, EHD3, HER1, NMA111, PBP4, MFB1, IKI3, NDL1, SUT433, YOR238W, SUT750, QDR2, RDI1, SUT014, CUT437, MSC6, SUT497, YCR051W, MRPL33, RPL14A, TRM7, RNH202, RTC5, SUT027, CDC5, SUT729, YOR131C, CUT665, GLG2, SUT268, SUT705, MED4, RCR2, EFB1, RXT2, KGD1, TUP1, RNH203, YDR338C, SED1, CUT522, HIS2, SUT145, MET17, APC4, NKP2, MKK2, NDC1, PET100, NIP7, VHT1, SUT685, BNI5, SNA3, EGH1, MRP4, POB3, PIB2, SUT317, and NTO1, were inactivated by gene deletion. Then, α-amylase activity was evaluated in the respective strains. The overexpression of *BNA7, SUT074, TOM22, TLG2, YDR262W, ALP1, ENO2*, *NMA2, PRY2,* and *INP51* were identified as preferred for the exemplary α-amylase secretion in the Ethanol Red^{®} strain.

In the context of the present invention, any suitable cell of a yeast or filamentous fungus can be used for the production of the protein of interest according to the present invention. Preferably, said yeast or filamentous fungal cell is selected from the group consisting of *Aspergillus* spp., *Trichoderma* spp., *Saccharomyces cerevisiae, Schizosaccharomyces pombe, Kluyveromyces* ssp., *Pichia* spp., *Hansenula polymorpha, Fusarium* spp., *Neurospora* spp., and *Penicillium* spp., preferably *Saccharomyces cerevisiae.*

In the yeast or filamentous fungal cell according to the present invention, the at least one fungal gene showing increased expression and/or overexpression and/or showing reduced expression and/or inactivation is a native gene and/or is a recombinant gene, i.e. a modified gene of the yeast or filamentous fungal cell itself, or at least one gene that is recombinantly introduced and may be a heterologous gene, i.e. coming from a different strain or fungal species. Preferably, the recombinant gene is integrated into the genome as an expression cassette. Respective expression cassettes for fungal expression are known, and basically consist of a promoter, the fungal gene, and a terminator. Alternatively or in additionally, the gene can be extrachromosomally expressed, preferably using a replicative expression vector, such as a shuttle vector. Promoters used in yeast and fungal expression systems are usually either inducible or constitutive.

The folding and glycosylation of the secretory proteins in the endoplasmatic reticulum (ENDR) is assisted by numerous ENDR-resident proteins. The chaperones like Bip (GRP78), GRP94 or yeast Lhs1p help the secretory protein to fold by binding to exposed hydrophobic regions in the unfolded states and preventing unfavourable interactions (Blond-Elguindi et al., 1993, Cell 75:717-728). The chaperones are also important for the translocation of the proteins through the ENDR membrane. The proteins like protein disulphide isomerase and its homologs and prolyl-peptidyl cis-trans isomerase assist in formation of disulphide bridges and formation of the right conformation of the peptide chain adjacent to proline residues, respectively. A machinery including many protein components also resides in the ENDR for the addition of the N-linked core glycans to the secretory protein and for the initial trimming steps of the glycans.

Preferred is therefore the yeast or filamentous fungal cell according to the present invention, wherein the cell furthermore comprises at least one additional recombinant secretion promoting gene, for example a fungal gene for a chaperone, for a foldase and/or for a glycosylation-promoting protein. Like the other genes as disclosed herein, these proteins may be controllably expressed, inducible, constitutive, and even overexpressed.

Therefore, preferred is the yeast or filamentous fungal cell according to the present invention, wherein the increased expression and/or overexpression or reduced expression and/or inactivation of the at least one fungal gene or the at least one additional recombinant secretion promoting gene is constitutive or inducible.

Another important aspect of the present invention relates to a method for producing a secreted protein in a yeast or filamentous fungal cell, comprising the steps of i) providing a yeast or filamentous fungal cell producing at least one secreted protein of interest according to the present invention as above, ii) suitably culturing said yeast or filamentous fungal cell in suitable culture medium, and iii) isolating said secreted protein from said culture medium. Methods for isolating proteins from cultures are known by the person of skill.

Culturing methods for producing proteins in yeast or filamentous fungal cells are known by the person of skill, and can be readily adjusted to the present invention. Culturing can be continuous or in batches or fed-batches. Preferred is the method according to the present invention, further comprising suitably inducing the increased expression and/or overexpression or reduced expression and/or inactivation of the at least one fungal gene. Induction can be achieved based on the promotor(s) as used, e.g. by adding inducers, or switching conditions, e.g. temperature.

There are many examples of engineering of *S. cerevisiae* for improved protein production, including optimizing of fermentation process, selecting the expression vectors systems, choosing the signal sequence for extracellular targeting and engineering host strains for better folding and post-translational modification (Tohda H., Kumagai H., Takegawa, K, (2010) Engineering of protein secretion in yeast: strategies and impact on protein production. Appl Microbiol Biotechnol 86: 403-417).

Preferred is the method according to the present invention, wherein about 30% or more or 40% or more, preferably about 50% or more, more preferably to about 75% or more of said at least one secreted protein is produced, when compared to the production of a control yeast or filamentous fungal cell, preferably one that does not contain a gene as mentioned above leading to increased secretion of the protein of interest

Another important aspect of the present invention relates to a method for producing a yeast or filamentous fungal cell producing at least one secreted protein of interest, comprising introducing into said cell producing at least one secreted protein of interest at least one fungal gene selected from the group consisting of MIC19, TOM22, NKP1, DML1, CUT859, GAL80, APM3, COQ10, BLM10, MDH1, VHS2, ASA1, TRP4, YPS7, CUT824, YOR318C, PRM7, ERV46, FIT2, GPM3, CUT892, SRN2, SUT643, CUT461, THR4, GMH1, SOL1, NAB6, YPR148C, ALP1, CUT097, ATG33, YOR316C-A, SOG2, MCM6, SUT230, SUT419, TIF11, TAF5, PHO91, AIM32, ENO2, UBA2, PUS5, ERG1, SUT311, KSS1, MRP10, CUT598, CUT188, YOR238W, EMW1, BNA7, SNR63, CCT3, PRY2, MAL11, KRS1 RAI1, SUT784, YPR148C, YEL1, CUT832, NMA2, VPS27, SUT428, PEX29, YLR446W, WBP1, AVT2, CUT854, TRM10, SLX9, YPL077C, PET122, TFG2, PUN1, CUT152, AIR2, CUT571, RPS26B, RRT6, RPC19, URA3, YGR045C, SMC3, PNG1, THI6, MEU1, CUT239, NSE4, SUT074, AAH1, RMD5, CUT607, ACS1, MNN1, ARH1, YHR140W, CET1, RRB1, YLR342W-A, RPS22B, CHS5, YIL165C, SUT093, LPX1, NCA3, EFG1, NBP35, CUT765, MSL1, SCD6, ATG42, CHS6, COQ2, RPO31, MKK1, HED1, PBP2, BET5, CUT678, YGR021W, SUT474, YGL159W, IRC21, VHR1, SPP1, PRP43, ZRT1, YLR041W, SUT711, COX18, CBP6, SUT575, CLG1, CUT213, QCR10, SNR3, MSS2, CUT505, YOS1, SUT073, UTP21, ACA1, CUT632, RIP1, HUL5, CUT727, RPL35B, CUT184, CUT420, YFL041W-A, SUT460, ATG10, MFA1, UGX2, TRK2, CUT704, SUT083, TRE1, RVS161, LEA1, EBP2, THI80, CTI6, CUT322, XPT1, MRPL35, YPL025C, SUT737, PGA2, ULP2, MRX16, EST1, NUP100, IES3, ATG39, YMR084W, SUT428, YPL119C-A, MIN8, CUT490, SUT287, KEL3, SUT678, SEC3, SOL4, SIS2, CUT915, RRP3, ESA1, PCL8, TRX3, YKL115C, EMP65, ZDS1, CUT167, SOD1, UBR2, LSP1, SNR81, RGD3, YTP1, SMY2, CUT449, FIN1, YKL106C-A, YAR019W-A, CCH1, AYR1, SUT573, VNX1, FOL3, SUT511, GIS4, CUT743, RPL24A, HMT1, SUT333, SPP2, SUT128, SMC6, PHR1, RPS15, CUT642, GYP7, tK(CUU)K, CUT896, SLM5, CUT586, CUT158, and RRP12, wherein said at least one fungal gene shows increased expression and/or overexpression, and/or wherein said cell comprises at least one fungal gene selected from the group consisting of TLG2, CUT901, ATG33, THR4, YDR262W, CMC1, MRP17, YPT52, CUT312, MRPS5, RDR1, DAL7, RPL20A, YBR137W, RPL36B, YEL008C-A, RAX1, INP51, CUT729, UBP8, CUT258, YLR342W-A, SUT568, PEX7, MSD1, CUT136, TIM10, CUT361, snR51, TAL1, RIP1, MRP10, SUT078, MRP51, GLO3, EHD3, HER1, NMA111, PBP4, MFB1, IKI3, NDL1, SUT433, YOR238W, SUT750, QDR2, RDI1, SUT014, CUT437, MSC6, SUT497, YCR051W, MRPL33, RPL14A, TRM7, RNH202, RTC5, SUT027, CDC5, SUT729, YOR131C, CUT665, GLG2, SUT268, SUT705, MED4, RCR2, EFB1, RXT2, KGD1, TUP1, RNH203, YDR338C, SED1, CUT522, HIS2, SUT145, MET17, APC4, NKP2, MKK2, NDC1, PET100, NIP7, VHT1, SUT685, BNI5, SNA3, EGH1, MRP4, POB3, PIB2, SUT317, and NTO1, wherein said at least one fungal gene shows reduced expression and/or inactivation. Preferably, said at least one fungal gene is integrated into the genome as an expression cassette and/or extrachromosomally expressed, preferably using a replicative expression vector.

Preferred is a method of the present invention for producing a yeast or filamentous fungal cell producing at least one secreted protein of interest according to the present invention, comprising introducing into said cell producing at least one secreted protein of interest at least one fungal gene selected from the group consisting of MIC19, TOM22, NKP1, DML1, CUT859, GAL80, APM3, COQ10, BLM10, MDH1, EMW1, BNA7, SNR63, CCT3, PRY2, MAL11, KRS1, RAI1, SUT784, YPR148C, YEL1, CUT832, NMA2, VPS27, SUT428, PEX29, YLR446W, and WBP1, wherein said at least one fungal gene shows increased expression and/or overexpression, and/or wherein said cell comprises at least one fungal gene selected from the group consisting of TLG2, CUT901, ATG33, THR4, NDC1, PET100, NIP7, VHT1, and SUT685, wherein said at least one fungal gene shows reduced expression and/or inactivation.

Furthermore, the method according to the invention may include further introducing into said cell a fungal gene selected from the group consisting of THR4, MRP10, RIP1, YLR342W-A, ATG33, and YOR238W, either showing an increased expression and/or overexpression or reduced expression and/or inactivation, depending on the experimental conditions.

In a preferred embodiment according to the method according to the present invention, said method further comprises introducing into said cell the fungal gene *PDI1,* showing an increased expression and/or overexpression. Preferably, said at least one fungal gene is also integrated into the genome as an expression cassette and/or extrachromosomally expressed, preferably using a replicative expression vector

Finally, another important aspect of the present invention relates to the use of a yeast or filamentous fungal cell according to the present invention for producing at least one secreted protein of interest, preferably using a method according to the present invention.

In summary, the present invention provides the following items.

Item 1. A yeast or filamentous fungal cell producing at least one secreted protein of interest, wherein said cell comprises at least one fungal gene selected from the group consisting of MIC19, TOM22, NKP1, DML1, CUT859, GAL80, APM3, COQ10, BLM10, MDH1, VHS2, ASA1, TRP4, YPS7, CUT824, YOR318C, PRM7, ERV46, FIT2, GPM3, CUT892, SRN2, SUT643, CUT461, THR4, GMH1, SOL1, NAB6, YPR148C, ALP1, CUT097, ATG33, YOR316C-A, SOG2, MCM6, SUT230, SUT419, TIF11, TAF5, PHO91, AIM32, ENO2, UBA2, PUS5, ERG1, SUT311, KSS1, MRP10, CUT598, CUT188, YOR238W, EMW1, BNA7, SNR63, CCT3, PRY2, MAL11, KRS1 RAI1, SUT784, YPR148C, YEL1, CUT832, NMA2, VPS27, SUT428, PEX29, YLR446W, WBP1, AVT2, CUT854, TRM10, SLX9, YPL077C, PET122, TFG2, PUN1, CUT152, AIR2, CUT571, RPS26B, RRT6, RPC19, URA3, YGR045C, SMC3, PNG1, THI6, MEU1, CUT239, NSE4, SUT074, AAH1, RMD5, CUT607, ACS1, MNN1, ARH1, YHR140W, CET1, RRB1, YLR342W-A, RPS22B, CHS5, YIL165C, SUT093, LPX1, NCA3, EFG1, NBP35, CUT765, MSL1, SCD6, ATG42, CHS6, COQ2, RPO31, MKK1, HED1, PBP2, BET5, CUT678, YGR021W, SUT474, YGL159W, IRC21, VHR1, SPP1, PRP43, ZRT1, YLR041W, SUT711, COX18, CBP6, SUT575, CLG1, CUT213, QCR10, SNR3, MSS2, CUT505, YOS1, SUT073, UTP21, ACA1, CUT632, RIP1, HUL5, CUT727, RPL35B, CUT184, CUT420, YFL041W-A, SUT460, ATG10, MFA1, UGX2, TRK2, CUT704, SUT083, TRE1, RVS161, LEA1, EBP2, THI80, CTI6, CUT322, XPT1, MRPL35, YPL025C, SUT737, PGA2, ULP2, MRX16, EST1, NUP100, IES3, ATG39, YMR084W, SUT428, YPL119C-A, MIN8, CUT490, SUT287, KEL3, SUT678, SEC3, SOL4, SIS2, CUT915, RRP3, ESA1, PCL8, TRX3, YKL115C, EMP65, ZDS1, CUT167, SOD1, UBR2, LSP1, SNR81, RGD3, YTP1, SMY2, CUT449, FIN1, YKL106C-A, YAR019W-A, CCH1, AYR1, SUT573, VNX1, FOL3, SUT511, GIS4, CUT743, RPL24A, HMT1, SUT333, SPP2, SUT128, SMC6, PHR1, RPS15, CUT642, GYP7, tK(CUU)K, CUT896, SLM5, CUT586, CUT158, and RRP12, wherein said at least one fungal gene shows increased expression and/or overexpression, and/or wherein said cell comprises at least one fungal gene selected from the group consisting of TLG2, CUT901, ATG33, THR4, YDR262W, CMC1, MRP17, YPT52, CUT312, MRPS5, RDR1, DAL7, RPL20A, YBR137W, RPL36B, YEL008C-A, RAX1, INP51, CUT729, UBP8, CUT258, YLR342W-A, SUT568, PEX7, MSD1, CUT136, TIM10, CUT361, snR51, TAL1, RIP1, MRP10, SUT078, MRP51, GLO3, EHD3, HER1, NMA111, PBP4, MFB1, IKI3, NDL1, SUT433, YOR238W, SUT750, QDR2, RDI1, SUT014, CUT437, MSC6, SUT497, YCR051W, MRPL33, RPL14A, TRM7, RNH202, RTC5, SUT027, CDC5, SUT729, YOR131C, CUT665, GLG2, SUT268, SUT705, MED4, RCR2, EFB1, RXT2, KGD1, TUP1, RNH203, YDR338C, SED1, CUT522, HIS2, SUT145, MET17, APC4, NKP2, MKK2, NDC1, PET100, NIP7, VHT1, SUT685, BNI5, SNA3, EGH1, MRP4, POB3, PIB2, SUT317, and NTO1, wherein said at least one fungal gene shows reduced expression and/or inactivation.

Item 2. The yeast or filamentous fungal cell according to Item 1, wherein said cell comprises at least one fungal gene selected from the group consisting of MIC19, TOM22, NKP1, DML1, CUT859, GAL80, APM3, COQ10, BLM10, MDH1, EMW1, BNA7, SNR63, CCT3, PRY2, MAL11, KRS1, RAI1, SUT784, YPR148C, YEL1, CUT832, NMA2, VPS27, SUT428, PEX29, YLR446W, and WBP1, wherein said at least one fungal gene shows increased expression and/or overexpression, and/or wherein said cell comprises at least one fungal gene selected from the group consisting of TLG2, CUT901, ATG33, THR4, NDC1, PET100, NIP7, VHT1, and SUT685, wherein said at least one fungal gene shows reduced expression and/or inactivation.

Item 3. The yeast or filamentous fungal cell according to Item 1 or 2, wherein said genes or SUTs or CUTs are furthermore selected from the group of genes or SUTs or CUTs having a value of logFC/FDR of more than 200, more preferred of more than 300, and most preferred of more than 500, based on the values as determined herein.

Item 4. The yeast or filamentous fungal cell according to any one of Items 1 to 3, further comprising a fungal gene selected from the group consisting of THR4, MRP10, RIP1, YLR342W-A, ATG33, and YOR238W, either showing an increased expression and/or overexpression or reduced expression and/or inactivation, depending on the experimental conditions.

Item 5. The yeast or filamentous fungal cell according to any one of Items 1 to 4, further comprising the fungal gene *PDI1,* showing an increased expression and/or overexpression.

Item 6. The yeast or filamentous fungal cell according to any one of Items 1 to 5, wherein said yeast or filamentous fungal cell is selected from the group consisting of *Aspergillus* spp., *Trichoderma* spp., *Saccharomyces cerevisiae, Schizosaccharomyces pombe, Kluyveromyces* ssp., *Pichia* spp., *Hansenula polymorpha, Fusarium* spp., *Neurospora* spp., and *Penicillium* spp., preferably *Saccharomyces cerevisiae.*

Item 7. The yeast or filamentous fungal cell according to any one of Items 1 to 6, wherein said at least one secreted protein of interest also shows an increased expression and/or overexpression.

Item 8. The yeast or filamentous fungal cell according to any one of Items 1 to 7, wherein said at least one fungal gene showing increased expression and/or overexpression and/or showing reduced expression and/or inactivation is a native gene and/or is a recombinant gene, wherein preferably said recombinant gene is integrated into the genome as an expression cassette and/or extrachromosomally expressed, preferably using a replicative expression vector.

Item 9. The yeast or filamentous fungal cell according to any one of Items 1 to 8, wherein the cell furthermore comprises at least one additional recombinant secretion promoting gene, for example a gene for a chaperone, for a foldase and/or for a glycosylation-promoting protein.

Item 10. The yeast or filamentous fungal cell according to any one of Items 1 to 9, wherein the increased expression and/or overexpression or reduced expression and/or inactivation of the at least one fungal gene or the at least one additional recombinant secretion promoting gene is constitutive or inducible.

Item 11. The yeast or filamentous fungal cell according to any one of Items 1 to 10, wherein the cell produces the at least one secreted protein to about 30% or more, or to about 40% or more, preferably about 50% or more, more preferably to about 75% or more, when compared to a control yeast or filamentous fungal cell.

Item 12. A method for producing a secreted protein in a yeast or filamentous fungal cell, comprising the steps of i) providing a yeast or filamentous fungal cell producing at least one secreted protein of interest according to any one of Items 1 to 11, ii) culturing said yeast or filamentous fungal cell in suitable culture medium, and iii) isolating said secreted protein from said culture medium.

Item 13. The method according to Item 12, further comprising suitably inducing the increased expression and/or overexpression or reduced expression and/or inactivation of the at least one fungal gene.

Item 14. The method according to Item 11 or 12, wherein about 30% or more, or about 40% or more, preferably about 50% or more, more preferably to about 75% or more of said at least one secreted protein is produced, when compared to the production of a control yeast or filamentous fungal cell.

Item 15. A method for producing a yeast or filamentous fungal cell producing at least one secreted protein of interest, comprising introducing into said cell producing at least one secreted protein of interest at least one fungal gene selected from the group consisting of MIC19, TOM22, NKP1, DML1, CUT859, GAL80, APM3, COQ10, BLM10, MDH1, EMW1, BNA7, SNR63, CCT3, PRY2, MAL11, KRS1, RAI1, SUT784, YPR148C, YEL1, CUT832, NMA2, VPS27, SUT428, PEX29, YLR446W, and WBP1, wherein said at least one fungal gene shows increased expression and/or overexpression, and/or wherein said cell comprises at least one fungal gene selected from the group consisting of TLG2, CUT901, ATG33, THR4, NDC1, PET100, NIP7, VHT1, and SUT685, wherein said at least one fungal gene shows reduced expression and/or inactivation.

Item 16. The method according to Item 15, further introducing into said cell a fungal gene selected from the group consisting of THR4, MRP10, RIP1, YLR342W-A, ATG33, and YOR238W, either showing an increased expression and/or overexpression or reduced expression and/or inactivation, depending on the experimental conditions.

Item 17. The method according to Item 15 or 16, further introducing into said cell the fungal gene *PDI1,* showing an increased expression and/or overexpression.

Item 18. The method according to any one of Items 15 to 17, wherein said at least one fungal gene is integrated into the genome as an expression cassette and/or extrachromosomally expressed, preferably using a replicative expression vector.

Item 19. Use of a yeast or filamentous fungal cell according to any one of Items 1 to 10 for producing at least one secreted protein of interest.

The present invention will now be described further in the following examples with reference to the accompanying Figure, nevertheless, without being limited thereto. For the purposes of the present invention, all references as cited herein are incorporated by reference in their entireties.

Figure 1 shows the map of plasmid pLI410-062 as used in the methods according to the present invention.

Figure 2 A and B shows the results of the α-amylase secretion measurements relative to baseline for selected genes of the present invention as box plots in % control over time (4, 24, 48, and 120 hours). Genes are ALP1, BNA7, GMH1, SUT074, TFG2, ENO2, NMA2, PRY2, and TOM22. HAC1 is control.

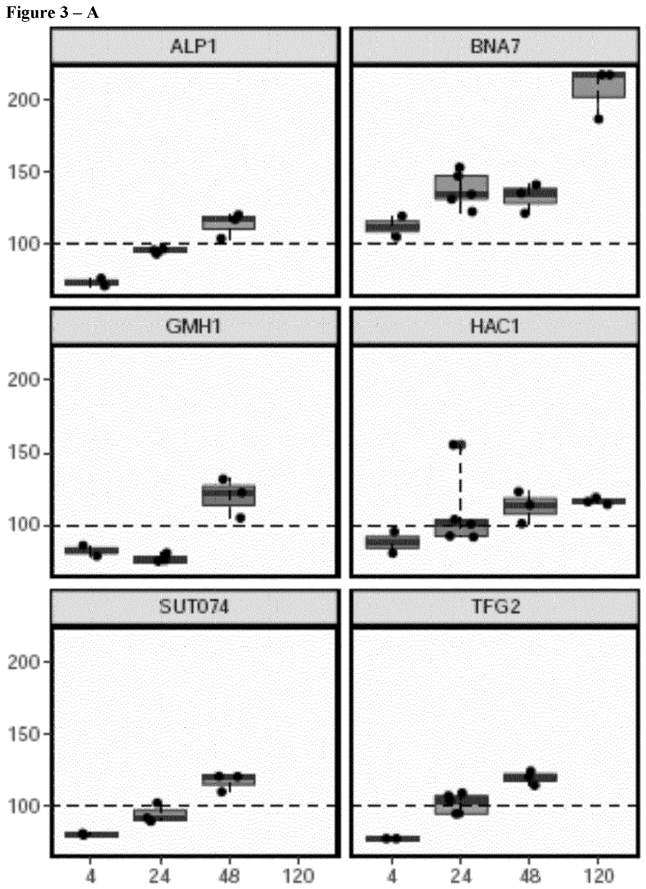

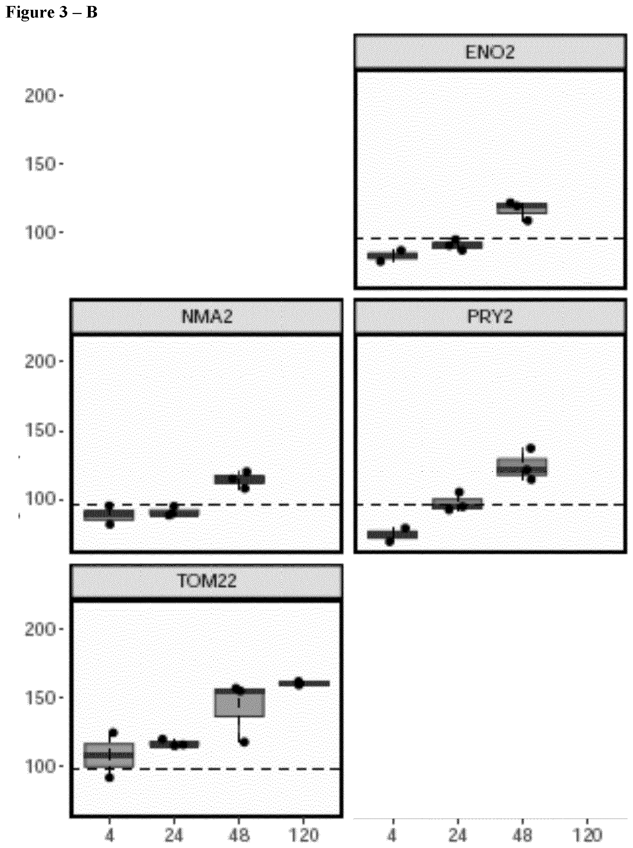

Figure 3 A and B shows the results of the α-amylase secretion measurements per cell for selected genes of the present invention as box plots in % control over time (4, 24, 48, and 120 hours). Genes are ALP1, BNA7, GMH1, SUT074, TFG2, ENO2, NMA2, PRY2, and TOM22. HAC1 is control.

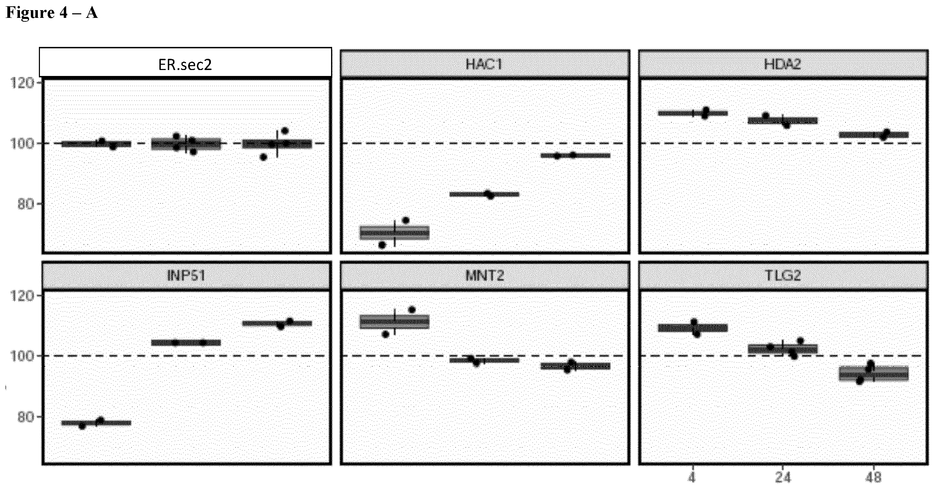

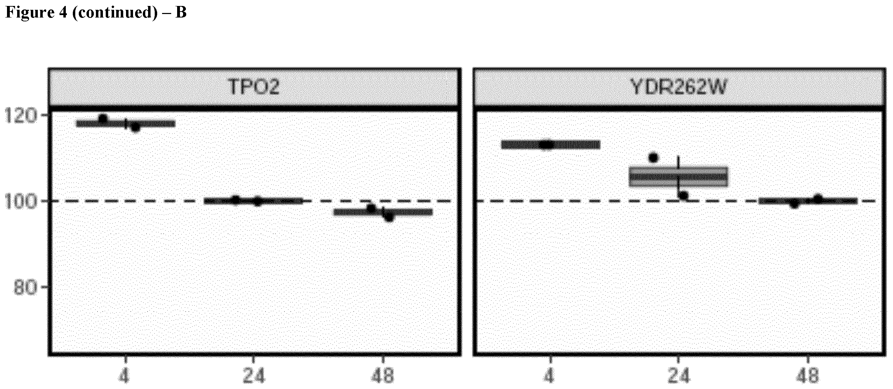

Figure 4 A and B shows the results of the α-amylase secretion measurements (total amylase) for selected genes of the present invention as box plots in % control over time (4, 24, and 48 hours). Genes are INP51, MNT2, TLG2, TPO2, and YDR262W. HAC1, HDA2 and ER.sec2 are controls.

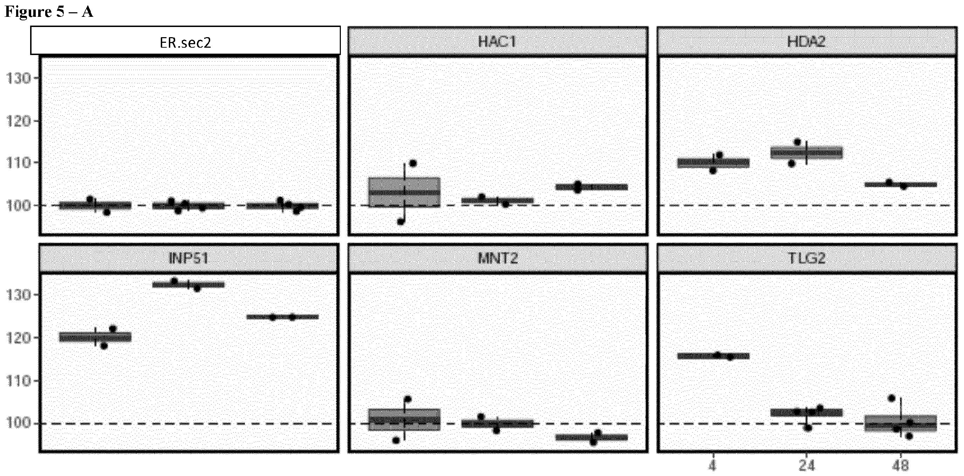

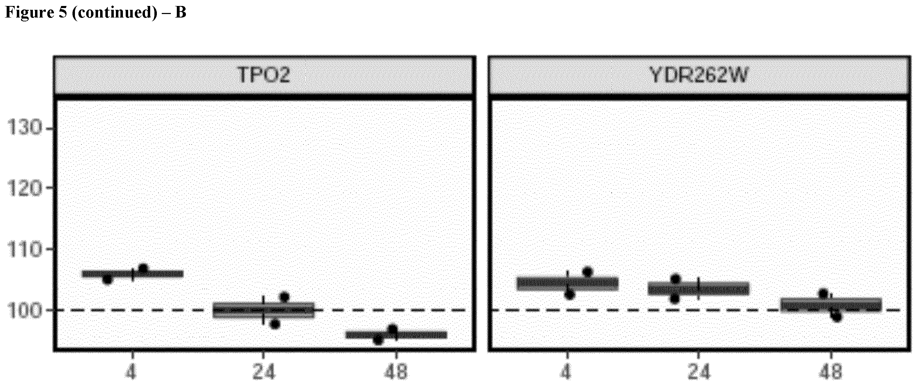

Figure 5 A and B shows the results of the α-amylase secretion measurements per cell for selected genes of the present invention as box plots in % control overtime (4, 24, and 48 hours). Genes are INP51, MNT2, TLG2, TPO2, and YDR262W. HAC1, HDA2 and ER.sec2 are controls.

### Examples

### Materials and Methods

### Construction of yeast overexpression strains

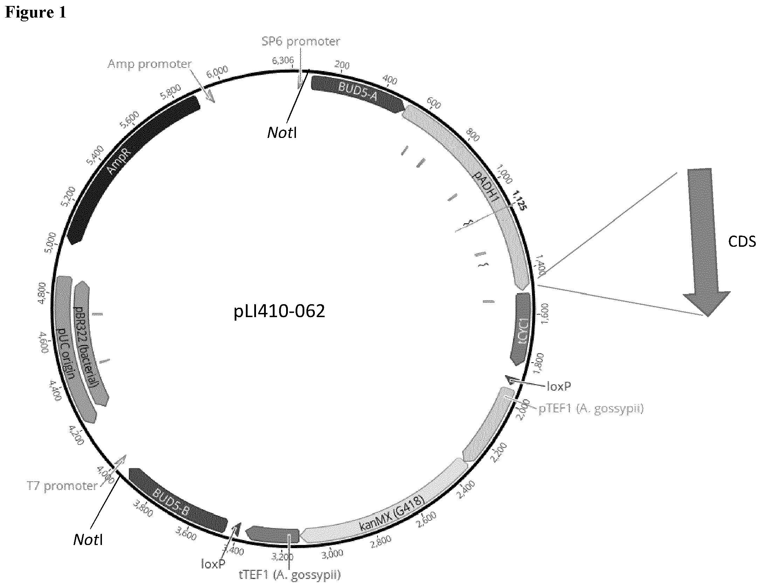

For overexpression of target genes using genome integration, candidate genes were cloned into plasmid pLI410-062 between the *Asc*I and *Sbf*I restriction sites, which was then linearized by NotI enzyme, and transformed into yeast strain ER.sec2. The plasmid integrates into the yeast chromosome at the *BUD5* locus (Figure 1). For plasmid based overexpression of target genes, native candidate genes were cloned into plasmid p427-TEF between SpeI and SalI and transformed into yeast strain ER.sec2.

### Construction of yeast deletion strains

Deletion strains were constructed by golden gate assembly of annealed oligos with gRNA sequences targeting the start and end position of the target gene, into sgRNA expression vector pWS082. The assembled plasmid and Cas9 expression vector pWS173 were linearized using EcoRV or BsmBI and co-transformed with annealed repair fragments, consisting of the joined 60 bp flanking regions of each target gene, which upon successful homology directed repair, resulted in the deletion of the target gene in ER.sec2.

### α-Amylase activity measurement

Preculture of YPD was performed, either with 22h of culture on SD-2xSCAA, or 22h and 96 h of culture on YPD. SD-2×SCAA medium was prepared as described previously (Hackel et al. 2006; Tyo et al. 2012), and the composition of SD-2xSCAA was as follows: 10 g/L glucose, 6.7 g/L yeast nitrogen base without amino acids, 2 g/L, KH2PO4 (pH 6.0 by NaOH), and 1 g/L BSA, containing filter sterilized SCAA solution (190 mg/L arginine, 108 mg/L methionine, 52 mg/L tyrosine, 290 mg/L isoleucine, 440 mg/L lysine, 200 mg/L phenylalanine, 1,260 mg/L, glutamic acid, 400 mg/L aspartic acid, 380 mg/L valine, 220 mg/L threonine, 130 mg/L glycine, 400 mg/L leucine, 40 mg/L tryptophan, and 140 mg/L histidine) (see Liu et al., 2013 - Correlation of cell growth and heterologous protein production by *Saccharomyces cerevisiae).*

The initial OD₆₀₀ₙₘ was 0.1, and flasks of 250 ml + 50 ml of medium were used. Culture density was measured at OD₆₀₀ₙₘ.

For the assay, 100 µL of supernatant + 900 µL of acetate buffer 50mM pH5.5 were combined, and 10 µL of sample were incubated for 5 min at 40°C in a PCR well plate.

Afterwards, 10 µL of BPNPG7 substrate was added, followed by incubation for 10 min at 40°C. The reaction was stopped by adding 150 µL of Trizma base 1%, followed by vortexing. The result was read at an OD of 400nm, which generally required a prior step of 10 or 20-fold dilution.

### Calculation of α-amylase activity

The activity U was calculated as U = (ΔE₄₀₀/10) × (0.17/0.01) × (1/18.1) × D

E₄₀₀ : Sample absorbance - blank absorbance, 10 : time of reaction, 0.17 : total volume of reaction, 0.01 : volume of sample, 18.1 : E_{mM} p-nitrophenol in Trizma base 1%, D : Dilution of sample. Normalization of α-amylase activity was performed with respective OD₆₀₀ₙₘ.

### Results

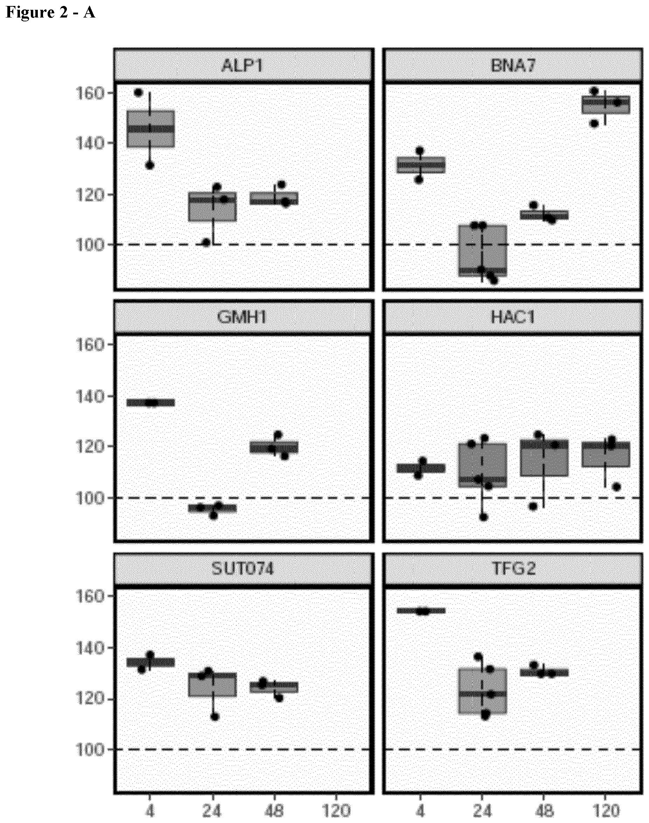

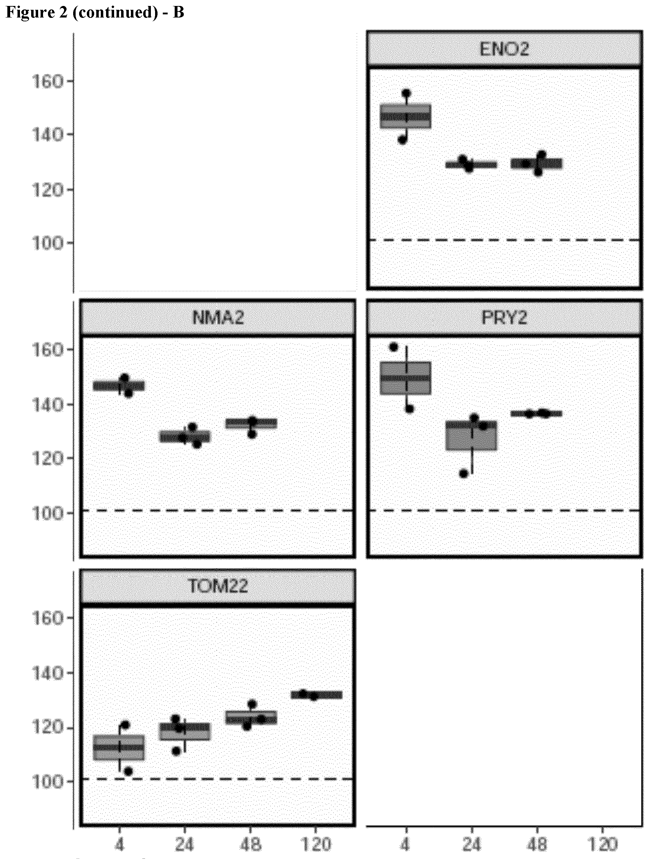

The following genes and SUTs (stable uncharacterized transcripts) or CUTs (cryptic unstable transcripts) were identified as being of relevance, and relevance was defined as at least 2% increase of amylase activity (see above). See also Figure 2.

1. Genes that were activated/overexpressed (integration of overexpression cassette into the genome and/or overexpression through a replicative plasmid) after statistical and enrichment analysis - preferred selection. logFC (log fold change) indicates the measure of enrichment, a higher value, equals a higher enrichment in the experiments as performed. FDR (false discovery rate) indicates the corrected p-value, a lower value means less variance between replicates as performed.

| Gene (common name, SUT or CUT or systematic designation) | Name and function (if known) | logFC | FDR |
|---|---|---|---|
| MIC19 | Component of the MICOS complex | 13.883 | 0.036 |
| TOM22 | Translocase of the Outer Mitochondrial membrane; responsible for initial import of mitochondrially directed proteins | 13.781 | 0.008 |
| NKP1 | Non-essential Kinetochore Protein | 13.389 | 0.012 |
| DML1 | Drosophila melanogaster Misato-Like protein, Essential protein involved in mtDNA inheritance | 13.307 | 0.014 |
| CUT859 | SUT or CUT | 13.152 | 0.033 |
| GAL80 | GALactose metabolism, Transcriptional regulator involved in the repression of GAL genes | 12.170 | 0.008 |
| APM3 | clathrin Adaptor Protein complex Medium chain | 12.088 | 0.020 |
| COQ10 | COenzyme Q, Coenzyme Q (ubiquinone) binding protein | 12.048 | 0.025 |
| BLM10 | BLeoMycin resistance, Proteasome activator | 12.008 | 0.030 |
| MDH1 | Malate DeHydrogenase, Mitochondrial malate dehydrogenase | 11.915 | 0.008 |
| VHS2 | Viable in a Hal3 Sit4 background, Regulator of septin dynamics | 11.838 | 0.032 |
| ASA1 | AStra Associated protein, Subunit of the ASTRA complex | 11.801 | 0.015 |
| TRP4 | TRyPtophan, Anthranilate phosphoribosyl transferase | 11.698 | 0.019 |
| YPS7 | YaPSin, Putative GPI-anchored aspartic protease | 11.620 | 0.030 |
| CUT824 | SUT or CUT | 11.529 | 0.041 |
| YOR318C | Gene of unknown function | 11.515 | 0.013 |
| PRM7 | Pheromone-Regulated Membrane protein | 11.485 | 0.023 |
| ERV46 | ER Vesicle, Protein localized to COPII-coated vesicles | 11.350 | 0.010 |
| FIT2 | Facilitator of Iron Transport, Mannoprotein that is incorporated into the cell wall | 11.287 | 0.034 |
| GPM3 | Glycerate PhosphoMutase | 11.062 | 0.019 |
| CUT892 | SUT or CUT | 10.972 | 0.050 |
| SRN2 | Suppressor of Rna mutations, Number 2 | 10.938 | 0.021 |
| SUT643 | SUT or CUT | 10.910 | 0.039 |
| CUT461 | SUT or CUT | 10.901 | 0.042 |
| THR4 | THReonine requiring, Threonine synthase | 10.840 | 0.047 |
| GMH1 | Gea1-6 Membrane-associated High-copy suppressor; Golgi membrane protein of unknown function | 10.780 | 0.055 |
| SOL1 | Suppressor Of Los1-1, Protein with a possible role in tRNA export | 10.725 | 0.026 |
| NAB6 | Nucleic Acid Binding protein, Putative RNA-binding protein | 10.674 | 0.013 |
| YPR148C | Gene of unknown function | 10.614 | 0.027 |
| ALP1 | Arginine transporter | 10.598 | 0.046 |
| CUT097 | SUT or CUT | 10.597 | 0.046 |
| ATG33 | AuTophaGy related, Mitochondrial mitophagy-specific protein | 10.585 | 0.030 |
| YOR316C-A | Gene of unknown function | 10.547 | 0.025 |
| SOG2 | Key component of the RAM signaling network; required for proper cell morphogenesis and cell separation after mitosis | 10.546 | 0.039 |
| MCM6 | MiniChromosome Maintenance, Protein involved in DNA replication | 10.531 | 0.019 |
| SUT230 | SUT or CUT | 10.507 | 0.010 |
| SUT419 | SUT or CUT | 10.398 | 0.027 |
| TIF11 | Translation Initiation Factor | 10.334 | 0.024 |
| TAF5 | TATA binding protein-Associated Factor, involved in RNA polymerase II transcription initiation and in chromatin modification | 10.328 | 0.027 |
| PHO91 | PHOsphate metabolism, Low-affinity vacuolar phosphate transporter | 10.303 | 0.024 |
| AIM32 | Altered Inheritance rate of Mitochondria, 2Fe-2S mitochondrial protein involved in redox quality control | 10.271 | 0.042 |
| ENO2 | ENOlase, Enolase II, a phosphopyruvate hydratase | 10.260 | 0.050 |
| UBA2 | UBiquitin Activating, Subunit of heterodimeric nuclear SUMO activating enzyme E1 with Aos1p | 10.215 | 0.030 |
| PUS5 | PseudoUridine Synthase | 10.197 | 0.030 |
| ERG1 | ERGosterol biosynthesis, Squalene epoxidase | 10.139 | 0.013 |
| SUT311 | SUT or CUT | 10.130 | 0.012 |
| KSS1 | Kinase Suppressor of Sst2 mutations, Mitogen-activated protein kinase (MAPK) | 10.116 | 0.039 |
| MRP10 | Mitochondrial Ribosomal Protein, Mitochondrial ribosomal protein of the small subunit | 10.099 | 0.023 |
| CUT598 | SUT or CUT | 10.099 | 0.046 |
| CUT188 | SUT or CUT | 10.073 | 0.026 |
| YOR238W | Gene of unknown function | 10.023 | 0.025 |
| EMW1 | Essential for Maintenance of the cell Wall, Essential conserved protein with a role in cell wall integrity | 15.549 | 0.071 |
| BNA7 | Biosynthesis of NAD, Formylkynurenine formamidase | 14.863 | 0.071 |
| SNR63 | Small Nucleolar RNA, C/D box small nucleolar RNA (snoRNA) | 14.717 | 0.071 |
| CCT3 | Chaperonin Containing TCP-1, Subunit of the cytosolic chaperonin Cct ring complex | 14.647 | 0.071 |
| PRY2 | Pathogen Related in Yeast, Sterol binding protein involved in the export of acetylated sterols | 14.548 | 0.071 |
| MAL11 | MALtose fermentation, High-affinity maltose transporter (alpha-glucoside transporter) | 14.484 | 0.071 |
| KRS1 | Lysyl (K) tRNA Synthetase | 14.290 | 0.072 |
| RAI1 | Rat1p Interacting Protein, Nuclear decapping endonuclease | 14.254 | 0.071 |
| SUT784 | SUT or CUT | 13.682 | 0.071 |
| YPR148C | Gene of unknown function | 13.572 | 0.071 |
| YEL1 | Yeast EFA6-Like, Guanine nucleotide exchange factor specific for Arf3p | 13.417 | 0.096 |
| CUT832 | SUT or CUT | 13.118 | 0.071 |
| NMA2 | Nicotinamide Mononucleotide Adenylyltransferase | 13.116 | 0.071 |
| VPS27 | Vacuolar Protein Sorting, Endosomal protein that forms a complex with Hse1p | 12.963 | 0.071 |
| SUT428 | SUT or CUT | 12.841 | 0.089 |
| PEX29 | PEroXisome related, ER-resident protein involved in peroxisomal biogenesis | 12.477 | 0.071 |
| YLR446W | Gene of unknown function | 12.369 | 0.071 |
| WBP1 | Wheat germ agglutinin-Binding Protein, Beta subunit of the oligosaccharyl transferase glycoprotein complex | 12.078 | 0.087 |
| AVT2 | Amino acid Vacuolar Transport, Putative transporter | 10.965 | 0.071 |
| CUT854 | SUT or CUT | 10.873 | 0.093 |
| TRM10 | Transfer RNA Methyltransferase, methylates the N-1 position of guanine at position 9 in tRNAs | 10.442 | 0.099 |
| SLX9 | Protein required for pre-rRNA processing | 9.996 | 0.012 |
| YPL077C | Gene of unknown function | 9.994 | 0.038 |
| PET122 | PETite colonies, Mitochondrial translational activator specific for the COX3 mRNA | 9.982 | 0.039 |
| TFG2 | Transcription Factor G; involved in both transcription initiation and elongation of RNA polymerase II | 9.973 | 0.090 |
| PUN1 | Plasma membrane protein Upregulated during Nitrogen stress | 9.950 | 0.027 |
| CUT152 | SUT or CUT | 9.936 | 0.020 |
| AIR2 | Arginine methyltransferase-Interacting RING finger protein, involved in nuclear RNA processing and degradation | 9.886 | 0.044 |
| CUT571 | SUT or CUT | 9.799 | 0.033 |
| RPS26B | Protein component of the small (40S) ribosomal subunit | 9.789 | 0.023 |
| RRT6 | Regulator of rDNA Transcription | 9.749 | 0.012 |
| RPC19 | RNA Polymerase C, RNA polymerase subunit AC19 | 9.715 | 0.047 |
| URA3 | URAcil requiring, Orotidine-5'-phosphate (OMP) decarboxylase | 9.687 | 0.046 |
| YGR045C | Gene of unknown function | 9.679 | 0.039 |
| SMC3 | Stability of MiniChromosomes, Subunit of the multiprotein cohesin complex | 9.669 | 0.025 |
| PNG1 | Peptide N-Glycanase | 9.654 | 0.019 |
| THI6 | THIamine biosynthesis, Thiamine-phosphate diphosphorylase and hydroxyethylthiazole kinase | 9.653 | 0.033 |
| MEU1 | Multicopy Enhancer of UAS2, Methylthioadenosine phosphorylase (MTAP) | 9.558 | 0.031 |
| CUT239 | SUT or CUT | 9.531 | 0.032 |
| NSE4 | Non-SMC Element, Component of the SMC5-SMC6 complex | 9.502 | 0.023 |
| SUT074 | SUT or CUT | 9.478 | 0.019 |
| AAH1 | Adenine AminoHydrolase, Adenine deaminase (adenine aminohydrolase) | 9.454 | 0.044 |
| RMD5 | Required for Meiotic nuclear Division, Component of GID Complex that confers ubiquitin ligase (U3) activity | 9.452 | 0.024 |
| CUT607 | SUT or CUT | 9.313 | 0.020 |
| ACS1 | Acetyl CoA Synthetase, Acetyl-coA synthetase isoform | 9.305 | 0.036 |
| MNN1 | MaNNosyltransferase, Alpha-1,3-mannosyltransferase | 9.265 | 0.019 |
| ARH1 | Adrenodoxin Reductase Homolog, Oxidoreductase of the mitochondrial inner membrane | 9.244 | 0.039 |
| YHR140W | Gene of unknown function | 9.220 | 0.021 |
| CET1 | Capping Enzyme Triphosphatase, RNA 5'-triphosphatase involved in mRNA 5' capping | 9.203 | 0.019 |
| RRB1 | Regulator of Ribosome Biogenesis, Specific assembly chaperone for ribosomal protein Rpl3p | 9.185 | 0.030 |
| YLR342W-A | Gene of unknown function | 9.166 | 0.010 |
| RPS22B | Ribosomal Protein of the Small subunit, Protein component of the small (40S) ribosomal subunit | 9.154 | 0.024 |
| CHS5 | CHitin Synthase-related, Component of the exomer complex | 9.143 | 0.027 |
| YIL165C | Gene of unknown function | 9.140 | 0.040 |
| SUT093 | SUT or CUT | 9.139 | 0.030 |
| LPX1 | Lipase of PeroXisomes, Peroxisomal matrix-localized lipase | 9.114 | 0.039 |
| NCA3 | Nuclear Control of ATPase, Protein involved in mitochondrion organization | 9.078 | 0.026 |
| EFG1 | Exit From G1, Ribosome biogenesis factor required for maturation of 18S rRNA | 9.063 | 0.040 |
| NBP35 | Nucleotide Binding Protein, Essential cytoplasmic iron-sulfur cluster binding protein | 9.055 | 0.042 |
| CUT765 | SUT or CUT | 9.038 | 0.037 |
| MSL1 | MUD Synthetic Lethal | 9.019 | 0.015 |
| SCD6 | Suppressor of Clathrin Deficiency, Repressor of translation initiation | 9.004 | 0.025 |
| ATG42 | AuTophaGy, Vacuolar serine-type carboxypeptidase | 9.001 | 0.028 |
| CHS6 | CHitin Synthase-related, Member of the ChAPs (Chs5p-Arflp-binding proteins) family | 8.974 | 0.020 |
| COQ2 | COenzyme Q, Para hydroxybenzoate polyprenyl transferase | 8.973 | 0.045 |
| RPO31 | RNA Polymerase, RNA polymerase III largest subunit C160 | 8.969 | 0.044 |
| MKK1 | Mitogen-activated protein Kinase-Kinase, MAPKK involved in the protein kinase C signaling pathway | 8.958 | 0.030 |
| HED1 | High copy suppressor of rED1, Meiosis-specific protein | 8.903 | 0.025 |
| PBP2 | Pbp1p Binding Protein, RNA binding protein; has similarity to mammalian heterogeneous nuclear RNP K protein | 8.891 | 0.027 |
| BET5 | Blocked Early in Transport, Core component of transport protein particle (TRAPP) complexes I-III | 8.890 | 0.019 |
| CUT678 | SUT or CUT | 8.876 | 0.045 |
| YGR021W | Gene of unknown function | 8.823 | 0.012 |
| SUT474 | SUT or CUT | 8.811 | 0.042 |
| YGL159W | Gene of unknown function | 8.802 | 0.014 |
| IRC21 | Increased Recombination Centers, unknown function | 8.795 | 0.027 |
| VHR1 | VHt1 Regulator, Transcriptional activator | 8.760 | 0.046 |
| SPP1 | Setlc, Phd finger Protein, Subunit of COMPASS (Set1C) | 8.721 | 0.025 |
| PRP43 | Pre-mRNA Processing, RNA helicase in the DEAH-box family | 8.707 | 0.042 |
| ZRT1 | Zinc-Regulated Transporter, High-affinity zinc transporter of the plasma membrane; responsible for the majority of zinc uptake | 8.705 | 0.039 |
| YLR041W | Gene of unknown function | 8.687 | 0.044 |
| SUT711 | SUT or CUT | 8.686 | 0.039 |
| COX18 | Cytochrome c OXidase, Protein required for membrane insertion of C-terminus of Cox2p | 8.685 | 0.046 |
| CBP6 | Cytochrome B Protein synthesis, Mitochondrial protein required for translation of the COB mRNA | 8.678 | 0.043 |
| SUT575 | SUT or CUT | 8.651 | 0.042 |
| CLG1 | Cyclin-Like Gene, Cyclin-like protein that interacts with Pho85p | 8.651 | 0.047 |
| CUT213 | SUT or CUT | 8.610 | 0.036 |
| QCR10 | ubiQuinol-cytochrome C oxidoReductase, Subunit of the ubiqunol-cytochrome c oxidoreductase complex | 8.604 | 0.019 |
| SNR3 | Small Nucleolar RNA, H/ACA box small nucleolar RNA (snoRNA) | 8.571 | 0.044 |
| MSS2 | Mitochondrial Splicing, Peripherally bound inner membrane protein of the mitochondrial matrix | 8.559 | 0.023 |
| CUT505 | SUT or CUT | 8.557 | 0.039 |
| YOS1 | Yip One Suppressor, Integral membrane protein required for ER to Golgi transport | 8.540 | 0.023 |
| SUT073 | SUT or CUT | 8.519 | 0.033 |
| UTP21 | U Three Protein, Subunit of U3-containing 90S preribosome and SSU processome complexes | 8.511 | 0.039 |
| | | | |
| ACA1 | ATF/CREB Activator, ATF/CREB family basic leucine zipper (bZIP) transcription factor | 8.478 | 0.045 |
| CUT632 | SUT or CUT | 8.475 | 0.039 |
| RIP1 | Rieske Iron-sulfur Protein, Ubiquinol-cytochrome-c reductase | 8.466 | 0.037 |
| HUL5 | Hect Ubiquitin Ligase, Multiubiquitin chain assembly factor (E4) | 8.383 | 0.042 |
| CUT727 | SUT or CUT | 8.373 | 0.030 |
| RPL35B | Ribosomal 60S subunit protein L35B | 8.360 | 0.019 |
| CUT 184 | SUT or CUT | 8.304 | 0.039 |
| CUT420 | SUT or CUT | 8.300 | 0.023 |
| YFL041W-A | Gene of unknown function | 8.290 | 0.010 |
| SUT460 | SUT or CUT | 8.248 | 0.023 |
| ATG10 | AuTophaGy related, Conserved E2-like conjugating enzyme | 8.244 | 0.019 |
| MFA1 | Mating Factor A, Mating pheromone α-factor | 8.231 | 0.023 |
| UGX2 | Protein of unknown function | 8.226 | 0.023 |
| TRK2 | TRansport of potassium (K), Component of the Trklp-Trk2p potassium transport system | 8.218 | 0.027 |
| CUT704 | SUT or CUT | 8.201 | 0.041 |
| SUT083 | SUT or CUT | 8.189 | 0.025 |
| TRE1 | Transferrin REceptor like, Transferrin receptor-like protein | 8.183 | 0.046 |
| RVS161 | Reduced Viability on Starvation, Amphiphysin-like lipid raft protein | 8.110 | 0.034 |
| LEA1 | Looks Exceptionally like U2A, Component of U2 snRNP complex | 8.092 | 0.044 |
| EBP2 | EBNA1-binding protein (homolog), Required for 25S rRNA maturation and 60S ribosomal subunit assembly; | 8.089 | 0.030 |
| THI80 | THIamine metabolism, Thiamine pyrophosphokinase | 8.071 | 0.012 |
| CTI6 | Cyc8-Tup1 Interacting protein, Component of the Rpd3L histone deacetylase complex | 8.065 | 0.019 |
| CUT322 | SUT or CUT | 8.002 | 0.027 |
| XPT1 | Xanthine Phosphoribosyl Transferase, Xanthine-guanine phosphoribosyl transferase | 7.984 | 0.036 |
| MRPL35 | Mitochondrial Ribosomal Protein, Large subunit | 7.963 | 0.031 |
| YPL025C | Gene of unknown function | 7.962 | 0.037 |
| SUT737 | SUT or CUT | 7.950 | 0.025 |
| PGA2 | Processing of Gas1p and ALP, Essential protein required for maturation of Gas1p and Pho8p | 7.941 | 0.046 |
| ULP2 | UbL-specific Protease, Peptidase that deconjugates Smt3/SUMO-1 peptides from proteins | 7.935 | 0.033 |
| MRX16 | Mitochondrial oRganization of gene eXpression (MIOREX), Protein that associates with the large mitoribosomal subunit | 7.917 | 0.044 |
| EST1 | Ever Shorter Telomeres, TLC1 RNA-associated factor involved in telomere length regulation | 7.911 | 0.042 |
| NUP100 | NUclear Pore, FG-nucleoporin component of central core of the nuclear pore complex | 7.902 | 0.021 |
| IES3 | Ino Eighty Subunit, Subunit of the INO80 chromatin remodeling complex | 7.880 | 0.031 |
| ATG39 | AuTophaGy related, Autophagy receptor with a role in degradation of the ER and nucleus | 7.876 | 0.038 |
| YMR084W | Gene of unknown function | 7.850 | 0.027 |
| SUT428 | SUT or CUT | 7.827 | 0.030 |
| YPL119C-A | Gene of unknown function | 7.791 | 0.031 |
| MIN8 | mitochondrial MINi protein of 8 kDa | 7.783 | 0.027 |
| CUT490 | SUT or CUT | 7.779 | 0.045 |
| SUT287 | SUT or CUT | 7.708 | 0.027 |
| KEL3 | KELch | 7.705 | 0.027 |
| SUT678 | SUT or CUT | 7.699 | 0.025 |
| SEC3 | SECretory, Subunit of the exocyst complex | 7.691 | 0.045 |
| SOL4 | Suppressor Of Los1-1, 6-phosphogluconolactonase | 7.678 | 0.030 |
| SIS2 | SIt4 Suppressor, Negative regulatory subunit of protein phosphatase 1 (Ppz1p) | 7.650 | 0.026 |
| CUT915 | SUT or CUT | 7.649 | 0.044 |
| RRP3 | Ribosomal RNA Processing, Protein involved in rRNA processing | 7.635 | 0.034 |
| ESA1 | Catalytic subunit of the histone acetyltransferase complex (NuA4) | 7.612 | 0.031 |
| PCL8 | Pho85 CycLin, Cyclin | 7.581 | 0.046 |
| TRX3 | ThioRedoXin, Mitochondrial thioredoxin | 7.579 | 0.033 |
| YKL115C | Gene of unknown function | 7.530 | 0.043 |
| EMP65 | ER Membrane Protein of 65 kDa, Integral membrane protein of the ER | 7.520 | 0.029 |
| ZDS1 | Zillion Different Screens, Protein with a role in regulating Swe1p-dependent polarized growth | 7.488 | 0.049 |
| CUT167 | SUT or CUT | 7.486 | 0.016 |
| SOD1 | SuperOxide Dismutase, Cytosolic copper-zinc superoxide dismutase | 7.471 | 0.019 |
| UBR2 | Cytoplasmic ubiquitin-protein ligase (E3 | 7.470 | 0.044 |
| LSP1 | Long chain bases Stimulate Phosphorylation, Eisosome core component | 7.391 | 0.031 |
| SNR81 | H/ACA box small nucleolar RNA (snoRNA) | 7.389 | 0.030 |
| RGD3 | GTPase activating protein (GAP) for Rho3p | 7.370 | 0.032 |
| YTP1 | Yeast putative Transmembrane Protein, Probable type-III integral membrane protein of unknown function | 7.365 | 0.042 |
| SMY2 | Suppressor of MYo2-66, involved in COPII vesicle formation | 7.352 | 0.034 |
| CUT449 | SUT or CUT | 7.340 | 0.024 |
| FIN1 | Filaments In between Nuclei, Spindle pole body-related intermediate filament protein | 7.335 | 0.039 |
| YKL106C-A | Gene of unknown function | 7.293 | 0.021 |
| YAR019W-A | Gene of unknown function | 7.280 | 0.019 |
| CCH1 | Calcium Channel Homolog, Voltage-gated high-affinity calcium channel | 7.270 | 0.031 |
| AYR1 | 1-AcyldihYdroxyacetone-phosphate Reductase, ifunctional triacylglycerol lipase and 1-acyl DHAP reductase | 7.243 | 0.012 |
| SUT573 | SUT or CUT | 7.234 | 0.042 |
| VNX1 | Vacuolar Na+/H+ eXchanger, Calcium/H+ antiporter localized to the endoplasmic reticulum membrane | 7.232 | 0.010 |
| FOL3 | FOLic acid synthesis, Dihydrofolate synthetase | 7.215 | 0.032 |
| SUT511 | SUT or CUT | 7.212 | 0.026 |
| GIS4 | GIg1-2 Suppressor, proposed to be involved in the RAS/cAMP signaling pathway | 7.196 | 0.027 |
| CUT743 | SUT or CUT | 7.171 | 0.034 |
| RPL24A | Ribosomal 60S subunit protein L24A | 7.169 | 0.039 |
| HMT1 | HnRNP MethylTransferase, Nuclear SAM-dependent mono- and asymmetric methyltransferase | 7.163 | 0.026 |
| SUT333 | SUT or CUT | 7.141 | 0.031 |
| SPP2 | Suppressor of PrP, Essential protein that promotes the first step of splicing | 7.137 | 0.027 |
| SUT128 | SUT or CUT | 7.120 | 0.049 |
| SMC6 | Structural Maintenance of Chromosomes, Subunit of the SMC5-SMC6 complex | 7.120 | 0.047 |
| PHR1 | PHotoreactivation Repair deficient, DNA photolyase involved in photoreactivation | 7.119 | 0.030 |
| RPS15 | Protein component of the small (40S) ribosomal subunit | 7.072 | 0.012 |
| CUT642 | SUT or CUT | 7.066 | 0.025 |
| GYP7 | Gtpase-activating protein for Ypt7 Protein, GTPase-activating protein for yeast Rab family members | 7.063 | 0.021 |
| tK(CUU)K | Lysine tRNA (tRNA-Lys) | 7.034 | 0.041 |
| CUT896 | SUT or CUT | 7.026 | 0.041 |
| SLM5 | Synthetic Lethal with Mss4, Mitochondrial asparaginyl-tRNA synthetase | 7.024 | 0.039 |
| CUT586 | SUT or CUT | 7.020 | 0.038 |
| CUT158 | SUT or CUT | 7.003 | 0.030 |
| RRP12 | Ribosomal RNA Processing, Protein required for export of the ribosomal subunits | 7.002 | 0.031 |

1a. Gene to be preferably combined with the preferred selection

| | | | |
|---|---|---|---|
| PDI1 | Protein Disulfide Isomerase | 12.524 | 0.072 |

2. Genes or SUTs or CUTs that were inactivated/repressed after statistical and enrichment analysis - preferred selection. logFC (log fold change) indicates the measure of enrichment, a higher value, equals a higher enrichment in the experiments as performed. FDR (false discovery rate) indicates the corrected p-value, a lower value means less variance between replicates as performed.

| | | | |
|---|---|---|---|
| Gene (common name, SUT or CUT or systematic designation) | Name and function (if known) | logFC | FDR |
| TLG2 | T-snare affecting a Late Golgi compartment, Syntaxin-like t-SNARE | 13.51 | 0.010 |
| CUT901 | SUT or CUT | 11.72 | 0.009 |
| ATG33 | AuTophaGy related, Mitochondrial mitophagy-specific protein | 11.53 | 0.009 |
| THR4 | THReonine requiring, Threonine synthase | 11.49 | 0.009 |
| YDR262W | Gene of unknown function | 10.92 | 0.009 |
| CMC1 | Cx9C Mitochondrial protein necessary for full assembly of Cytochrome c oxidase, Copper-binding protein of the mitochondrial intermembrane space | 10.86 | 0.009 |
| MRP17 | Mitochondrial ribosomal protein of the small subunit | 10.20 | 0.019 |
| YPT52 | Yeast Protein Two, Endosomal Rab family GTPase; required for vacuolar protein sorting | 8.91 | 0.043 |
| CUT312 | SUT or CUT | 8.90 | 0.014 |
| MRPS5 | Mitochondrial Ribosomal Protein, Small subunit | 8.87 | 0.022 |
| RDR1 | Repressor of Drug Resistance, Transcriptional repressor involved in regulating multidrug resistance | 8.65 | 0.042 |
| DAL7 | Degradation of Allantoin, Malate synthase | 8.55 | 0.009 |
| RPL20A | Ribosomal 60S subunit protein L20A | 8.19 | 0.025 |
| YBR137W | Gene of unknown function | 8.11 | 0.056 |
| RPL36B | Ribosomal 60S subunit protein L36B | 8.02 | 0.028 |
| YEL008C-A | Gene of unknown function | 7.85 | 0.062 |
| RAX1 | Revert to Axial, Protein involved in establishing bud site selection | 7.65 | 0.019 |
| INP51 | INositol polyphosphate 5-Phosphatase | 7.51 | 0.102 |
| CUT729 | SUT or CUT | 7.27 | 0.066 |
| UBP8 | UBiquitin-specific processing Protease, Ubiquitin-specific protease component of the SAGA acetylation complex | 7.18 | 0.066 |
| CUT258 | SUT or CUT | 7.10 | 0.089 |
| YLR342W-A | Gene of unknown function | 7.09 | 0.025 |
| SUT568 | SUT or CUT | 7.04 | 0.027 |
| PEX7 | PEroXin, Peroxisomal signal receptor for peroxisomal matrix proteins | 7.00 | 0.024 |
| MSD1 | Mitochondrial aminoacyl-tRNA Synthetase, Aspartate (D) | 6.97 | 0.089 |
| CUT136 | SUT or CUT | 6.88 | 0.039 |
| TIM10 | Translocase of the Inner Membrane, Essential protein of the mitochondrial intermembrane space | 6.84 | 0.064 |
| CUT361 | SUT or CUT | 6.83 | 0.037 |
| snR51 | Small Nucleolar RNA | 6.80 | 0.085 |
| TAL1 | TransALdolase, Transaldolase, enzyme in the non-oxidative pentose phosphate pathway | 6.74 | 0.069 |
| RIP1 | Rieske Iron-sulfur Protein, Ubiquinol-cytochrome-c reductase | 6.65 | 0.058 |
| MRP10 | Mitochondrial Ribosomal Protein, Mitochondrial ribosomal protein of the small subunit | 6.63 | 0.051 |
| SUT078 | SUT or CUT | 6.52 | 0.074 |
| MRP51 | Mitochondrial Ribosomal Protein, Mitochondrial ribosomal protein of the small subunit | 6.51 | 0.065 |
| GLO3 | GLyOxalase, ADP-ribosylation factor GTPase activating protein (ARF GAP); involved in ER-Golgi transport | 6.51 | 0.053 |
| EHD3 | 3-hydroxyisobutyryl-CoA hydrolase | 6.50 | 0.025 |
| HER1 | Hmg2p ER Remodeling, Protein of unknown function | 6.48 | 0.051 |
| NMA111 | Nuclear Mediator of Apoptosis, Serine protease and general molecular chaperone | 6.45 | 0.041 |
| PBP4 | Pbp1p binding protein | 6.28 | 0.044 |
| MFB1 | Mitochondria-associated F-box protein; involved in maintenance of normal mitochondrial morphology | 6.25 | 0.098 |
| IKI3 | Insensitive to KIller toxin, Subunit of Elongator complex | 6.21 | 0.031 |
| NDL1 | NuDeL homolog, Homolog of nuclear distribution factor NudE | 6.15 | 0.057 |
| SUT433 | SUT or CUT | 5.99 | 0.022 |
| YOR238W | Gene of unknown function | 5.91 | 0.054 |
| SUT750 | SUT or CUT | 5.86 | 0.016 |
| QDR2 | QuiniDine Resistance, Plasma membrane transporter of the major facilitator superfamily | 5.84 | 0.020 |
| RDI1 | Rho GDP Dissociation Inhibitor | 5.79 | 0.023 |
| SUT014 | SUT or CUT | 5.76 | 0.059 |
| CUT437 | SUT or CUT | 5.75 | 0.045 |
| MSC6 | Meiotic Sister-Chromatid recombination, Multicopy suppressor of HER2 involved in mitochondrial translation | 5.66 | 0.055 |
| SUT497 | SUT or CUT | 5.54 | 0.072 |
| YCR051W | Gene of unknown function | 5.52 | 0.076 |
| MRPL33 | Mitochondrial Ribosomal Protein, Large subunit | 5.47 | 0.024 |
| RPL14A | Ribosomal 60S subunit protein L14A | 5.46 | 0.077 |
| TRM7 | 2'-O-ribose methyltransferase | 5.43 | 0.081 |
| RNH202 | Ribonuclease H2 subunit; required for RNase H2 activity | 5.43 | 0.083 |
| RTC5 | Restriction of Telomere Capping, Protein of unknown function | 5.38 | 0.060 |
| SUT027 | SUT or CUT | 5.34 | 0.058 |
| CDC5 | Cell Division Cycle, Polo-like kinase essential for mitotic cell cycle | 5.33 | 0.070 |
| SUT729 | SUT or CUT | 5.30 | 0.076 |
| YOR131C | Gene of unknown function | 5.28 | 0.078 |
| CUT665 | SUT or CUT | 5.12 | 0.097 |
| GLG2 | Glycogenin-Like Gene, Glycogenin glucosyltransferase | 5.12 | 0.079 |
| | | | |
| SUT268 | SUT or CUT | 4.89 | 0.087 |
| SUT705 | SUT or CUT | 4.87 | 0.086 |
| MED4 | MEDiator complex, Subunit of the RNA polymerase II mediator complex | 4.61 | 0.093 |
| RCR2 | Resistance to Congo Red, Vacuolar ubiquitin ligase-substrate adaptor | 4.59 | 0.055 |
| EFB1 | Elongation Factor Beta, Translation elongation factor 1 beta | 4.58 | 0.036 |
| RXT2 | Component of the histone deacetylase Rpd3L complex | 4.49 | 0.073 |
| KGD1 | alpha-KetoGlutarate Dehydrogenase, Subunit of the mitochondrial alpha-ketoglutarate dehydrogenase complex | 4.42 | 0.093 |
| TUP1 | dTMP-UPtake, General repressor of transcription | 4.35 | 0.080 |
| RNH203 | Ribonuclease H2 subunit | 4.31 | 0.096 |
| YDR338C | Gene of unknown function | 3.92 | 0.029 |
| SED1 | Suppression of Exponential Defect, Major stress-induced structural GPI-cell wall glycoprotein | 3.81 | 0.089 |
| CUT522 | SUT or CUT | 3.75 | 0.092 |
| HIS2 | HIStidine requiring, Histidinolphosphatase | 3.74 | 0.090 |
| SUT145 | SUT or CUT | 3.67 | 0.072 |
| MET17 | METhionine requiring, O-acetyl homoserine-O-acetyl serine sulfhydrylase | 3.58 | 0.063 |
| APC4 | Anaphase Promoting, Subunit of the Anaphase-Promoting Complex/Cyclosome (APC/C) | 3.58 | 0.077 |
| NKP2 | Non-essential Kinetochore Protein, Central kinetochore protein and subunit of the Ctf19 complex | 3.54 | 0.022 |
| MKK2 | Mitogen-activated Kinase Kinase, MAPKK involved in the protein kinase C signaling pathway | 3.05 | 0.042 |
| NDC1 | Nuclear Division Cycle, Subunit of the transmembrane ring of the nuclear pore complex (NPC) | 14.18 | 0.079 |
| PET100 | PETite colonies, Chaperone that facilitates the assembly of cytochrome c oxidase | 12.69 | 0.086 |
| NIP7 | Nuclear ImPort, Nucleolar protein required for 60S ribosome subunit biogenesis | 12.54 | 0.086 |
| VHT1 | Vitamin H Transporter, High-affinity plasma membrane H+-biotin (vitamin H) symporter | 12.31 | 0.086 |
| SUT685 | SUT or CUT | 12.07 | 0.086 |
| BNI5 | Bud Neck Involved, Linker protein responsible for recruitment of myosin to the bud neck | 11.96 | 0.086 |
| SNA3 | Sensitivity to NA+, Protein involved in efficient MVB sorting of proteins to the vacuole | 11.93 | 0.086 |
| EGH1 | Cryptococcus neoformans EGCrP2 Homolog, Steryl-beta-glucosidase with broad specificity for aglycones | 11.81 | 0.086 |
| MRP4 | Mitochondrial ribosomal protein of the small subunit | 11.67 | 0.086 |
| POB3 | POl1 Binding, Subunit of the heterodimeric FACT complex (Spt16p-Pob3p) | 10.87 | 0.086 |
| PIB2 | PtdIns(3)p-Binding, Phosphatidylinositol 3-phosphate binding protein | 10.80 | 0.086 |
| SUT317 | SUT or CUT | 10.74 | 0.086 |
| NTO1 | NuA Three Orf, Subunit of the NuA3 histone acetyltransferase complex | 10.62 | 0.086 |

3. Genes that were either overexpressed or inactivated/repressed depending on experimental conditions after statistical and enrichment analysis - preferred selection. logFC (log fold change) indicates the measure of enrichment, a higher value, equals a higher enrichment in the experiments as performed. FDR (false discovery rate) indicates the corrected p-value, a lower value means less variance between replicates as performed.

| Gene (common name, SUT or CUT or designation) | Name and function (if known) | logFC activation | logFC repression | FDR activation | FDR repression |
|---|---|---|---|---|---|
| THR4 | THReonine requiring, Threonine synthase | 10.84 | 11.49 | 0.047 | 0.009 |
| MRP10 | Mitochondrial Ribosomal Protein, Mitochondrial ribosomal protein of the small subunit | 10.10 | 6.63 | 0.023 | 0.051 |
| RIP1 | Rieske Iron-sulfur Protein, Ubiquinol-cytochrome-c reductase | 8.47 | 6.65 | 0.037 | 0.058 |
| YLR342W-A | Gene of unknown function | 9.17 | 7.09 | 0.010 | 0.025 |
| ATG33 | AuTophaGy related, Mitochondrial | 10.59 | 11.53 | 0.030 | 0.009 |
| | mitophagy-specific protein | | | | |
| YOR238W | Gene of unknown function | 10.02 | 5.91 | 0.025 | 0.054 |

3. Genes that were overexpressed - particularly preferred selection

| | | | |
|---|---|---|---|
| Gene (common name, SUT or CUT or systematic designation) | Name and function (if known) | logFC | FDR |
| MIC19 | Component of the MICOS complex | 13.883 | 0.036 |
| TOM22 | Translocase of the Outer Mitochondrial membrane; responsible for initial import of mitochondrially directed proteins | 13.781 | 0.008 |
| NKP1 | Non-essential Kinetochore Protein | 13.389 | 0.012 |
| DML1 | Drosophila melanogaster Misato-Like protein, Essential protein involved in mtDNA inheritance | 13.307 | 0.014 |
| CUT859 | SUT or CUT | 13.152 | 0.033 |
| GAL80 | GALactose metabolism, Transcriptional regulator involved in the repression of GAL genes | 12.170 | 0.008 |
| APM3 | clathrin Adaptor Protein complex Medium chain | 12.088 | 0.020 |
| COQ10 | COenzyme Q, Coenzyme Q (ubiquinone) binding protein | 12.048 | 0.025 |
| BLM10 | BLeoMycin resistance, Proteasome activator | 12.008 | 0.030 |
| MDH1 | Malate DeHydrogenase, Mitochondrial malate dehydrogenase | 11.915 | 0.008 |
| VHS2 | Viable in a Hal3 Sit4 background, Regulator of septin dynamics | 11.838 | 0.032 |
| ASA1 | AStra Associated protein, Subunit of the ASTRA complex | 11.801 | 0.015 |
| TRP4 | TRyPtophan, Anthranilate phosphoribosyl transferase | 11.698 | 0.019 |
| YPS7 | YaPSin, Putative GPI-anchored aspartic protease | 11.620 | 0.030 |
| CUT824 | SUT or CUT | 11.529 | 0.041 |
| YOR318C | Gene of unknown function | 11.515 | 0.013 |
| PRM7 | Pheromone-Regulated Membrane protein | 11.485 | 0.023 |
| ERV46 | ER Vesicle, Protein localized to COPII-coated vesicles | 11.350 | 0.010 |
| FIT2 | Facilitator of Iron Transport, Mannoprotein that is incorporated into the cell wall | 11.287 | 0.034 |
| GPM3 | Glycerate PhosphoMutase | 11.062 | 0.019 |
| CUT892 | SUT or CUT | 10.972 | 0.050 |
| SRN2 | Suppressor of Rna mutations, Number 2 | 10.938 | 0.021 |
| SUT643 | SUT or CUT | 10.910 | 0.039 |
| CUT461 | SUT or CUT | 10.901 | 0.042 |
| THR4 | THReonine requiring, Threonine synthase | 10.840 | 0.047 |
| GMH1 | Gea1-6 Membrane-associated High-copy suppressor; Golgi membrane protein of unknown function | 10.780 | 0.055 |
| SOL1 | Suppressor Of Los1-1, Protein with a possible role in tRNA export | 10.725 | 0.026 |
| NAB6 | Nucleic Acid Binding protein, Putative RNA-binding protein | 10.674 | 0.013 |
| YPR148C | Gene of unknown function | 10.614 | 0.027 |
| ALP1 | Arginine transporter | 10.598 | 0.046 |
| CUT097 | SUT or CUT | 10.597 | 0.046 |
| ATG33 | AuTophaGy related, Mitochondrial mitophagy-specific protein | 10.585 | 0.030 |
| YOR316C-A | Gene of unknown function | 10.547 | 0.025 |
| SOG2 | Key component of the RAM signaling network; required for proper cell morphogenesis and cell separation after mitosis | 10.546 | 0.039 |
| MCM6 | MiniChromosome Maintenance, Protein involved in DNA replication | 10.531 | 0.019 |
| SUT230 | SUT or CUT | 10.507 | 0.010 |
| SUT419 | SUT or CUT | 10.398 | 0.027 |
| TIF11 | Translation Initiation Factor | 10.334 | 0.024 |
| TAF5 | TATA binding protein-Associated Factor, involved in RNA polymerase II transcription initiation and in chromatin modification | 10.328 | 0.027 |
| PHO91 | PHOsphate metabolism, Low-affinity vacuolar phosphate transporter | 10.303 | 0.024 |
| AIM32 | Altered Inheritance rate of Mitochondria, 2Fe-2S mitochondrial protein involved in redox quality control | 10.271 | 0.042 |
| ENO2 | ENOlase, Enolase II, a phosphopyruvate hydratase | 10.260 | 0.050 |
| UBA2 | UBiquitin Activating, Subunit of heterodimeric nuclear SUMO activating enzyme E1 with Aos1p | 10.215 | 0.030 |
| PUS5 | PseudoUridine Synthase | 10.197 | 0.030 |
| ERG1 | ERGosterol biosynthesis, Squalene epoxidase | 10.139 | 0.013 |
| SUT311 | SUT or CUT | 10.130 | 0.012 |
| KSS1 | Kinase Suppressor of Sst2 mutations, Mitogen-activated protein kinase (MAPK) | 10.116 | 0.039 |
| MRP10 | Mitochondrial Ribosomal Protein, Mitochondrial ribosomal protein of the small subunit | 10.099 | 0.023 |
| CUT598 | SUT or CUT | 10.099 | 0.046 |
| CUT188 | SUT or CUT | 10.073 | 0.026 |
| YOR238W | Gene of unknown function | 10.023 | 0.025 |
| EMW1 | Essential for Maintenance of the cell Wall, Essential conserved protein with a role in cell wall integrity | 15.549 | 0.071 |
| BNA7 | Biosynthesis of NAD, Formylkynurenine formamidase | 14.863 | 0.071 |
| SNR63 | Small Nucleolar RNA, C/D box small nucleolar RNA (snoRNA) | 14.717 | 0.071 |
| CCT3 | Chaperonin Containing TCP-1, Subunit of the cytosolic chaperonin Cct ring complex | 14.647 | 0.071 |
| PRY2 | Pathogen Related in Yeast, Sterol binding protein involved in the export of acetylated sterols | 14.548 | 0.071 |
| MAL11 | MALtose fermentation, High-affinity maltose transporter (alpha-glucoside transporter) | 14.484 | 0.071 |
| KRS1 | Lysyl (K) tRNA Synthetase | 14.290 | 0.072 |
| RAI1 | Rat1p Interacting Protein, Nuclear decapping endonuclease | 14.254 | 0.071 |
| SUT784 | SUT or CUT | 13.682 | 0.071 |
| YPR148C | Gene of unknown function | 13.572 | 0.071 |
| YEL1 | Yeast EFA6-Like, Guanine nucleotide exchange factor specific for Arf3p | 13.417 | 0.096 |
| CUT832 | SUT or CUT | 13.118 | 0.071 |
| NMA2 | Nicotinamide Mononucleotide Adenylyltransferase | 13.116 | 0.071 |
| VPS27 | Vacuolar Protein Sorting, Endosomal protein that forms a complex with Hse1p | 12.963 | 0.071 |
| SUT428 | SUT or CUT | 12.841 | 0.089 |
| PEX29 | PEroXisome related, ER-resident protein involved in peroxisomal biogenesis | 12.477 | 0.071 |
| YLR446W | Gene of unknown function | 12.369 | 0.071 |
| WBP1 | Wheat germ agglutinin-Binding Protein, Beta subunit of the oligosaccharyl transferase glycoprotein complex | 12.078 | 0.087 |
| AVT2 | Amino acid Vacuolar Transport, Putative transporter | 10.965 | 0.071 |
| CUT854 | SUT or CUT | 10.873 | 0.093 |
| TRM10 | Transfer RNA Methyltransferase, methylates the N-1 position of guanine at position 9 in tRNAs | 10.442 | 0.099 |

Gene to be preferably combined with the particularly preferred selection

| | | | |
|---|---|---|---|
| PDI1 | Protein Disulfide Isomerase | 12.524 | 0.072 |

4. Genes or SUTs or CUTs that were inactivated/repressed after statistical and enrichment analysis - particularly preferred selection

| | | | |
|---|---|---|---|
| Gene (common name, SUT or CUT or systematic designation) | Name and function (if known) | logFC | FDR |
| TLG2 | T-snare affecting a Late Golgi compartment, Syntaxin-like t-SNARE | 13.51 | 0.010 |
| CUT901 | SUT or CUT | 11.72 | 0.009 |
| ATG33 | AuTophaGy related, Mitochondrial mitophagy-specific protein | 11.53 | 0.009 |
| THR4 | THReonine requiring, Threonine synthase | 11.49 | 0.009 |
| YDR262W | Gene of unknown function | 10.92 | 0.009 |
| CMC1 | Cx9C Mitochondrial protein necessary for full assembly of Cytochrome c oxidase, Copper-binding protein of the mitochondrial intermembrane space | 10.86 | 0.009 |
| MRP17 | Mitochondrial ribosomal protein of the small subunit | 10.20 | 0.019 |
| NDC1 | Nuclear Division Cycle, Subunit of the transmembrane ring of the nuclear pore complex (NPC) | 14.18 | 0.079 |
| PET100 | PETite colonies, Chaperone that facilitates the assembly of cytochrome c oxidase | 12.69 | 0.086 |
| NIP7 | Nuclear ImPort, Nucleolar protein required for 60S ribosome subunit biogenesis | 12.54 | 0.086 |
| VHT1 | Vitamin H Transporter, High-affinity plasma membrane H+-biotin (vitamin H) symporter | 12.31 | 0.086 |
| SUT685 | SUT or CUT | 12.07 | 0.086 |
| BNI5 | Bud Neck Involved, Linker protein responsible for recruitment of myosin to the bud neck | 11.96 | 0.086 |
| SNA3 | Sensitivity to NA+, Protein involved in efficient MVB sorting of proteins to the vacuole | 11.93 | 0.086 |
| EGH1 | Cryptococcus neoformans EGCrP2 Homolog, Steryl-beta-glucosidase with broad specificity for aglycones | 11.81 | 0.086 |
| MRP4 | Mitochondrial ribosomal protein of the small subunit | 11.67 | 0.086 |
| POB3 | POl1 Binding, Subunit of the heterodimeric FACT complex (Spt16p-Pob3p) | 10.87 | 0.086 |
| PIB2 | PtdIns(3)p-Binding, Phosphatidylinositol 3-phosphate binding protein | 10.80 | 0.086 |
| SUT317 | SUT or CUT | 10.74 | 0.086 |
| NTO1 | NuA Three Orf, Subunit of the NuA3 histone acetyltransferase complex | 10.62 | 0.086 |

5. Genes that were overexpressed - most preferred selection

| Gene (common name, SUT or CUT or systematic designation) | Name and function (if known) | logFC | FDR |
|---|---|---|---|
| MIC19 | Component of the MICOS complex | 13.883 | 0.036 |
| TOM22 | Translocase of the Outer Mitochondrial membrane; responsible for initial import of mitochondrially directed proteins | 13.781 | 0.008 |
| NKP1 | Non-essential Kinetochore Protein | 13.389 | 0.012 |
| DML1 | Drosophila melanogaster Misato-Like protein, Essential protein involved in mtDNA inheritance | 13.307 | 0.014 |
| CUT859 | SUT or CUT | 13.152 | 0.033 |
| GAL80 | GALactose metabolism, Transcriptional regulator involved in the repression of GAL genes | 12.170 | 0.008 |
| APM3 | clathrin Adaptor Protein complex Medium chain | 12.088 | 0.020 |
| COQ10 | COenzyme Q, Coenzyme Q (ubiquinone) binding protein | 12.048 | 0.025 |
| BLM10 | BLeoMycin resistance, Proteasome activator | 12.008 | 0.030 |
| MDH1 | Malate DeHydrogenase, Mitochondrial malate dehydrogenase | 11.915 | 0.008 |
| EMW1 | Essential for Maintenance of the cell Wall, Essential conserved protein with a role in cell wall integrity | 15.549 | 0.071 |
| BNA7 | Biosynthesis of NAD, Formylkynurenine formamidase | 14.863 | 0.071 |
| SNR63 | Small Nucleolar RNA, C/D box small nucleolar RNA (snoRNA) | 14.717 | 0.071 |
| CCT3 | Chaperonin Containing TCP-1, Subunit of the cytosolic chaperonin Cct ring complex | 14.647 | 0.071 |
| PRY2 | Pathogen Related in Yeast, Sterol binding protein involved in the export of acetylated sterols | 14.548 | 0.071 |
| MAL11 | MALtose fermentation, High-affinity maltose transporter (alpha-glucoside transporter) | 14.484 | 0.071 |
| KRS1 | Lysyl (K) tRNA Synthetase | 14.290 | 0.072 |
| RAI1 | Rat1p Interacting Protein, Nuclear decapping endonuclease | 14.254 | 0.071 |
| SUT784 | SUT or CUT | 13.682 | 0.071 |
| YPR148C | Gene of unknown function | 13.572 | 0.071 |
| YEL1 | Yeast EFA6-Like, Guanine nucleotide exchange factor specific for Arf3p | 13.417 | 0.096 |
| CUT832 | SUT or CUT | 13.118 | 0.071 |
| NMA2 | Nicotinamide Mononucleotide Adenylyltransferase | 13.116 | 0.071 |
| VPS27 | Vacuolar Protein Sorting, Endosomal protein that forms a complex with Hse1p | 12.963 | 0.071 |
| SUT428 | SUT or CUT | 12.841 | 0.089 |
| PEX29 | PEroXisome related, ER-resident protein involved in peroxisomal biogenesis | 12.477 | 0.071 |
| YLR446W | Gene of unknown function | 12.369 | 0.071 |
| WBP1 | Wheat germ agglutinin-Binding Protein, Beta subunit of the oligosaccharyl transferase glycoprotein complex | 12.078 | 0.087 |

Gene to be preferably combined with the most preferred selection

| | | | |
|---|---|---|---|
| PDI1 | Protein Disulfide Isomerase | 12.524 | 0.072 |

6. Genes or SUTs or CUTs that were inactivated/repressed after statistical and enrichment analysis - most preferred selection

| Gene (common name, SUT or CUT or systematic designation) | Name and function (if known) | logFC | FDR |
|---|---|---|---|
| TLG2 | T-snare affecting a Late Golgi compartment, Syntaxin-like t-SNARE | 13.51 | 0.010 |
| CUT901 | SUT or CUT | 11.72 | 0.009 |
| ATG33 | AuTophaGy related, Mitochondrial mitophagy-specific protein | 11.53 | 0.009 |
| THR4 | THReonine requiring, Threonine synthase | 11.49 | 0.009 |
| NDC1 | Nuclear Division Cycle, Subunit of the transmembrane ring of the nuclear pore complex (NPC) | 14.18 | 0.079 |
| PET100 | PETite colonies, Chaperone that facilitates the assembly of cytochrome c oxidase | 12.69 | 0.086 |
| NIP7 | Nuclear ImPort, Nucleolar protein required for 60S ribosome subunit biogenesis | 12.54 | 0.086 |
| VHT1 | Vitamin H Transporter, High-affinity plasma membrane H+-biotin (vitamin H) symporter | 12.31 | 0.086 |
| SUT685 | SUT or CUT | 12.07 | 0.086 |

Preferred are further genes or SUTs or CUTs that are selected from the group of genes or SUTs or CUTs having a value of logFC/FDR of more than 200, more preferred of more than 300, and most preferred of more than 500, based on the values herein.

### References as cited

1. Martinez Ruiz, J.; Liu, L.; Petranovic, D. (2012) "Pharmaceutical protein production by yeast: towards production of human blood proteins by microbial fermentation". Current Opinion in Biotechnology, vol. 23(6), pp. 965-971.
2. Falch EA. Industrial enzymes--developments in production and application. Biotechnol Adv. 1991;9(4):643-58. doi: 10.1016/0734-9750(91)90736-f. PMID: 14542053.
3. Demain AL, Vaishnav P. Production of recombinant proteins by microbes and higher organisms. Biotechnol Adv. 2009 May-Jun;27(3):297-306. doi: 10.1016/j.biotechadv.2009.01.008. Epub 2009 Jan 31. PMID: 19500547.
4. Zahrl RJ, Gasser B, Mattanovich D, Ferrer P. Detection and Elimination of Cellular Bottlenecks in Protein-Producing Yeasts. Methods Mol Biol. 2019;1923:75-95. doi: 10.1007/978-1-4939-9024-5_2. PMID: 30737735
5. Parapouli M, Vasileiadis A, Afendra AS, Hatziloukas E. Saccharomyces cerevisiae and its industrial applications. AIMS Microbiol. 2020 Feb 11;6(1):1-31. doi: 10.3934/microbiol.2020001. PMID: 32226912; PMCID: PMC7099199.
6. Dominguez AA, Lim WA, Qi LS. Beyond editing: repurposing CRISPR-Cas9 for precision genome regulation and interrogation. Nat Rev Mol Cell Biol. 2016 Jan;17(1):5-15. doi: 10.1038/nrm.2015.2. Epub 2015 Dec 16. PMID: 26670017; PMCID: PMC4922510.

## Claims

1. A yeast or filamentous fungal cell producing at least one secreted protein of interest, wherein said cell comprises at least one fungal gene selected from the group consisting of MIC19, TOM22, NKP1, DML1, CUT859, GAL80, APM3, COQ10, BLM10, MDH1, VHS2, ASA1, TRP4, YPS7, CUT824, YOR318C, PRM7, ERV46, FIT2, GPM3, CUT892, SRN2, SUT643, CUT461, THR4, GMH1, SOL1, NAB6, YPR148C, ALP1, CUT097, ATG33, YOR316C-A, SOG2, MCM6, SUT230, SUT419, TIF11, TAF5, PHO91, AIM32, ENO2, UBA2, PUS5, ERG1, SUT311, KSS1, MRP10, CUT598, CUT188, YOR238W, EMW1, BNA7, SNR63, CCT3, PRY2, MAL11, KRS1 RAI1, SUT784, YPR148C, YEL1, CUT832, NMA2, VPS27, SUT428, PEX29, YLR446W, WBP1, AVT2, CUT854, TRM10, SLX9, YPL077C, PET122, TFG2, PUN1, CUT152, AIR2, CUT571, RPS26B, RRT6, RPC19, URA3, YGR045C, SMC3, PNG1, THI6, MEU1, CUT239, NSE4, SUT074, AAH1, RMD5, CUT607, ACS1, MNN1, ARH1, YHR140W, CET1, RRB1, YLR342W-A, RPS22B, CHS5, YIL165C, SUT093, LPX1, NCA3, EFG1, NBP35, CUT765, MSL1, SCD6, ATG42, CHS6, COQ2, RPO31, MKK1, HED1, PBP2, BET5, CUT678, YGR021W, SUT474, YGL159W, IRC21, VHR1, SPP1, PRP43, ZRT1, YLR041W, SUT711, COX18, CBP6, SUT575, CLG1, CUT213, QCR10, SNR3, MSS2, CUT505, YOS1, SUT073, UTP21, ACA1, CUT632, RIP1, HUL5, CUT727, RPL35B, CUT184, CUT420, YFL041W-A, SUT460, ATG10, MFA1, UGX2, TRK2, CUT704, SUT083, TRE1, RVS161, LEA1, EBP2, THI80, CTI6, CUT322, XPT1, MRPL35, YPL025C, SUT737, PGA2, ULP2, MRX16, EST1, NUP100, IES3, ATG39, YMR084W, SUT428, YPL119C-A, MIN8, CUT490, SUT287, KEL3, SUT678, SEC3, SOL4, SIS2, CUT915, RRP3, ESA1, PCL8, TRX3, YKL115C, EMP65, ZDS1, CUT167, SOD1, UBR2, LSP1, SNR81, RGD3, YTP1, SMY2, CUT449, FIN1, YKL106C-A, YAR019W-A, CCH1, AYR1, SUT573, VNX1, FOL3, SUT511, GIS4, CUT743, RPL24A, HMT1, SUT333, SPP2, SUT128, SMC6, PHR1, RPS15, CUT642, GYP7, tK(CUU)K, CUT896, SLM5, CUT586, CUT158, and RRP12, wherein said at least one fungal gene shows increased expression and/or overexpression, and/or wherein said cell comprises at least one fungal gene selected from the group consisting of TLG2, CUT901, ATG33, THR4, YDR262W, CMC1, MRP17, YPT52, CUT312, MRPS5, RDR1, DAL7, RPL20A, YBR137W, RPL36B, YEL008C-A, RAX1, INP51, CUT729, UBP8, CUT258, YLR342W-A, SUT568, PEX7, MSD1, CUT136, TIM10, CUT361, snR51, TAL1, RIP1, MRP10, SUT078, MRP51, GLO3, EHD3, HER1, NMA111, PBP4, MFB1, IKI3, NDL1, SUT433, YOR238W, SUT750, QDR2, RDI1, SUT014, CUT437,
MSC6, SUT497, YCR051W, MRPL33, RPL14A, TRM7, RNH202, RTC5, SUT027, CDC5, SUT729, YOR131C, CUT665, GLG2, SUT268, SUT705, MED4, RCR2, EFB1, RXT2, KGD1, TUP1, RNH203, YDR338C, SED1, CUT522, HIS2, SUT145, MET17, APC4, NKP2, MKK2, NDC1, PET100, NIP7, VHT1, SUT685, BNI5, SNA3, EGH1, MRP4, POB3, PIB2, SUT317, and NTO1, wherein said at least one fungal gene shows reduced expression and/or inactivation, and optionally further comprising the fungal gene *PDI1,* showing an increased expression and/or overexpression.

2. The yeast or filamentous fungal cell according to claim 1, wherein said cell comprises at least one fungal gene selected from the group consisting of MIC19, TOM22, NKP1, DML1, CUT859, GAL80, APM3, COQ10, BLM10, MDH1, EMW1, BNA7, SNR63, CCT3, PRY2, MAL11, KRS1, RAI1, SUT784, YPR148C, YEL1, CUT832, NMA2, VPS27, SUT428, PEX29, YLR446W, and WBP1, wherein said at least one fungal gene shows increased expression and/or overexpression, and/or wherein said cell comprises at least one fungal gene selected from the group consisting of TLG2, CUT901, ATG33, THR4, NDC1, PET100, NIP7, VHT1, and SUT685, wherein said at least one fungal gene shows reduced expression and/or inactivation, and optionally further comprising the fungal gene *PDI1,* showing an increased expression and/or overexpression.

3. The yeast or filamentous fungal cell according to claim 1 or 2, wherein said genes or SUTs or CUTs are furthermore selected from the group of genes or SUTs or CUTs having a value of logFC/FDR of more than 200, more preferred of more than 300, and most preferred of more than 500, based on the values as determined herein.

4. The yeast or filamentous fungal cell according to any one of claims 1 to 3, further comprising a fungal gene selected from the group consisting of THR4, MRP10, RIP1, YLR342W-A, ATG33, and YOR238W, either showing an increased expression and/or overexpression or reduced expression and/or inactivation, depending on the experimental conditions.

5. The yeast or filamentous fungal cell according to any one of claims 1 to 4, wherein said yeast or filamentous fungal cell is selected from the group consisting of *Aspergillus* spp., *Trichoderma* spp., *Saccharomyces cerevisiae, Schizosaccharomyces pombe, Kluyveromyces* ssp., *Pichia* spp., *Hansenula polymorpha, Fusarium* spp., *Neurospora* spp., and *Penicillium* spp., preferably *Saccharomyces cerevisiae.*

6. The yeast or filamentous fungal cell according to any one of claims 1 to 5, wherein said at least one secreted protein of interest also shows an increased expression and/or overexpression.

7. The yeast or filamentous fungal cell according to any one of claims 1 to 6, wherein said at least one fungal gene showing increased expression and/or overexpression and/or showing reduced expression and/or inactivation is a native gene and/or is a recombinant gene, wherein preferably said recombinant gene is integrated into the genome as an expression cassette and/or extrachromosomally expressed, preferably using a replicative expression vector.

8. The yeast or filamentous fungal cell according to any one of claims 1 to 7, wherein the cell furthermore comprises at least one additional recombinant secretion promoting gene, for example a gene for a chaperone, for a foldase and/or for a glycosylation-promoting protein.

9. The yeast or filamentous fungal cell according to any one of claims 1 to 8, wherein the increased expression and/or overexpression or reduced expression and/or inactivation of the at least one fungal gene or the at least one additional recombinant secretion promoting gene is constitutive or inducible.

10. The yeast or filamentous fungal cell according to any one of claims 1 to 9, wherein the cell produces the at least one secreted protein to about 30% or more, or about 40% or more, preferably about 50% or more, more preferably to about 75% or more, when compared to a control yeast or filamentous fungal cell.

11. A method for producing a secreted protein in a yeast or filamentous fungal cell, comprising the steps of i) providing a yeast or filamentous fungal cell producing at least one secreted protein of interest according to any one of claims 1 to 10, ii) culturing said yeast or filamentous fungal cell in suitable culture medium, and iii) isolating said secreted protein from said culture medium, and optionally further comprising suitably inducing the increased expression and/or overexpression or reduced expression and/or inactivation of the at least one fungal gene.

12. The method according to claim 11, wherein preferably about 30% or more, or about 40% or more, preferably about 50% or more, more preferably to about 75% or more of said at least one secreted protein is produced, when compared to the production of a control yeast or filamentous fungal cell.

13. A method for producing a yeast or filamentous fungal cell producing at least one secreted protein of interest, comprising introducing into said cell producing at least one secreted protein of interest at least one fungal gene selected from the group consisting of MIC19, TOM22, NKP1, DML1, CUT859, GAL80, APM3, COQ10, BLM10, MDH1, EMW1, BNA7, SNR63, CCT3, PRY2, MAL11, KRS1, RAI1, SUT784, YPR148C, YEL1, CUT832, NMA2, VPS27, SUT428, PEX29, YLR446W, and WBP1, wherein said at least one fungal gene shows increased expression and/or overexpression, and/or wherein said cell comprises at least one fungal gene selected from the group consisting of TLG2, CUT901, ATG33, THR4, NDC1, PET100, NIP7, VHT1, and SUT685, wherein said at least one fungal gene shows reduced expression and/or inactivation, and optionally further introducing into said cell a fungal gene selected from the group consisting of THR4, MRP10, RIP1, YLR342W-A, ATG33, and YOR238W, either showing an increased expression and/or overexpression or reduced expression and/or inactivation, depending on the experimental conditions, and/or optionally further introducing into said cell the fungal gene *PDI1,* showing an increased expression and/or overexpression.

14. The method according to any one of claims 11 to 13, wherein said at least one fungal gene is integrated into the genome as an expression cassette and/or extrachromosomally expressed, preferably using a replicative expression vector.

15. Use of a yeast or filamentous fungal cell according to any one of claims 1 to 10 for producing at least one secreted protein of interest.
